(19) Europäisches Patentamt
European Patent Office
Office européen des brevets

(11) Publication number: **0 357 610 B1**

(12) # EUROPEAN PATENT SPECIFICATION

(45) Date of publication of patent specification: **16.09.92**

(51) Int. Cl.$^5$: **C07C 43/29**, C07C 43/257, C07C 43/20, C07C 43/168, C07C 43/174, C07C 323/00, C07D 213/64, C07D 317/46, C07D 307/78

(21) Application number: **88902034.3**

(22) Date of filing: **05.02.88**

(86) International application number: **PCT/US88/00346**

(87) International publication number: **WO 88/08416 (03.11.88 88/24)**

(54) **INSECTICIDAL CYCLOPROPYL-SUBSTITUTED DI(ARYL) COMPOUNDS.**

(30) Priority: **23.04.87 US 41551**
**09.09.87 US 94617**

(43) Date of publication of application: **14.03.90 Bulletin 90/11**

(45) Publication of the grant of the patent: **16.09.92 Bulletin 92/38**

(84) Designated Contracting States: **AT BE CH DE FR GB IT LI NL**

(56) References cited:
**EP-A- 0 094 085**
**WO-A-50/4651**
**GB-A- 2 085 006**
**US-A- 4 022 903**

(73) Proprietor: **FMC Corporation**
**2000 Market Street**
**Philadelphia Pennsylvania 19103(US)**

(72) Inventor: **MEIER, Gary, Allen**
**64 Wyndham Place, Robbinsville**
**Mercer County, NJ 08691(US)**
Inventor: **SIEBURTH, Scott, McNeill**
**263 Harrison Street, Princeton**
**Mercer County, NJ 08540(US)**
Inventor: **CULLEN, Thomas, Gerard**
**22 Harrison Avenue, Milltown**
**Middlesex County, NJ 08850(US)**
Inventor: **ENGEL, John, Francis**
**11 Lookout Lane, Washington Crossing**
**Bucks County, PA 18977(US)**

(74) Representative: **Vossius & Partner**
**Siebertstrasse 4 P.O. Box 86 07 67**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to novel pyrethroid-like insecticides which effectively control infestations of undesirable insects and acarids and simultaneously display remarkably low toxicity to fish. Synthetic pyrethroids have been the focus of intensive research activity for more than a decade. The pioneering work of Elliott, as described in U.S. 4,024,163, established that synthetic pyrethroids could be synthesized with sufficient stability to light to be commercially attractive. The vast majority of these new pyrethroids are esters of substituted cyclopropanecarboxylic acids similar to those described by Elliott. Initially, compounds having the aforementioned structure were thought to be required for insecticidal activity; however, considerable effort has been successfully directed toward defining compounds which are nominally described as pyrethroids based upon similarities in molecular geometry and insecticidal activity. In some of these compounds only the ester linkage has been retained; in others the substituted cyclopropane ring has been retained; and in yet others neither the substituted cyclopropane ring nor the ester linkage has been retained. In the current invention an unsubstituted cyclopropane group is incorporated into pyrethroid-like compounds. These novel compounds lack the substituted cyclopropanecarboxylic acid moiety typical of the compounds described by Elliott and those who followed him. Further, these compounds display pyrethroid-like insecticidal activity while possessing remarkably low toxicity to fish in comparison with the notorious toxicity to fish exhibited by cyclopropanecarboxylates.

United States Patent 4,397,864 discloses a class of pyrethroid-like compounds having the following subgeneric formula:

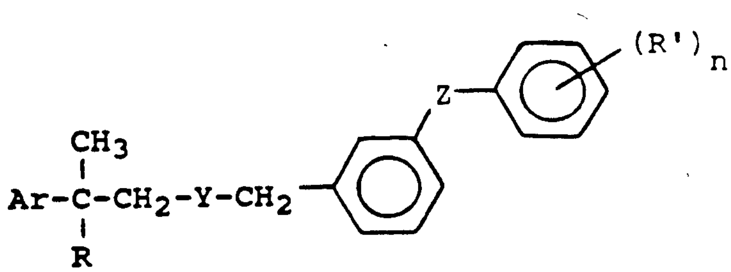

wherein

- Ar is optionally substituted phenyl, optionally substituted naphthyl, or 1,3-benzodioxol-5-yl;
- R is lower alkyl;
- Y is O or S;
- Z is O, S, or a carbonyl or methylene group;
- R' is H, F, lower alkyl, or lower alkoxy; and
- n is 1-5.

These compounds are alleged to have high insecticidal activity and low toxicity to fish.

United States Patent 4,073,812 covers a closely related series of compounds having the generic formula:

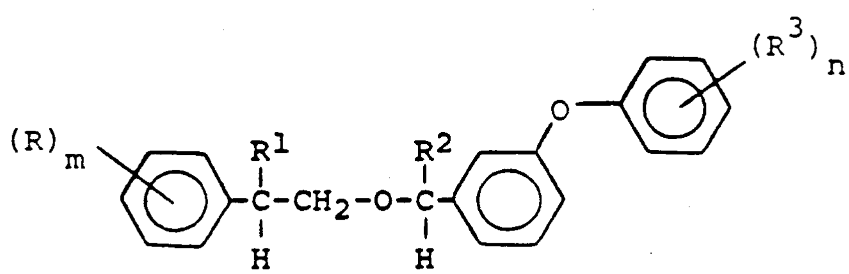

wherein

- R is halogen, lower alkyl, or lower alkoxy;
- m is 1 or 2;
- $R^1$ is branched chain alkyl of 3-6 carbon atoms;
- $R^2$ is hydrogen or alkynyl of 2-4 carbon atoms;
- $R^3$ is fluorine; and

n is 0 or 1.

In all examples $R^1$ is isopropyl. All compounds are asserted to be insecticidal, some more than others, but there is no indication or assertion about the degree of toxicity to fish.

United States Patent 4,562,213 covers another similar series of compounds of the formula:

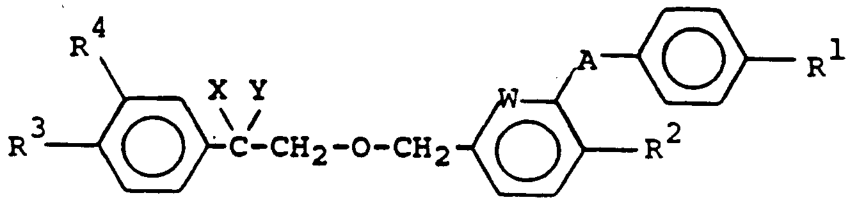

wherein

| | |
|---|---|
| $R^1$ | is hydrogen, halogen, or methyl; |
| $R^2$ | is hydrogen or fluorine; |
| W | is CH or N; |
| A | is oxygen, methylene, or imino; |
| X and Y | are both methyl or taken together form an optionally substituted cyclopropane ring; |
| $R^3$ and $R^4$ | may be the same or different and are hydrogen, halogen, lower alkyl, lower alkoxy, lower fluoroalkoxy, or taken together form a methylenedioxy bridge. |

In all cases where A is oxygen, X and Y are taken together to form a cyclopropane ring or a substituted cyclopropane ring. These compounds are asserted to be insecticidal and acaricidal without any assertion relating to fish toxicity.

United Kingdom patent application GB 2 120 664A discloses a class of aromatic-substituted alkane derivatives having the following generic formula:

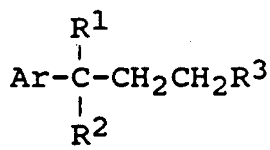

wherein

| | |
|---|---|
| Ar | stands for a substituted or unsubstituted phenyl or naphthyl group; |
| $R^1$ | stands for a methyl, ethyl, or isopropyl group and |
| $R^2$ | stands for a hydrogen atom or a methyl group or |
| $R^1$ and $R^2$ | taken together with the carbon to which they are attached represent a substituted or unsubstituted cycloalkyl group; and |
| $R^3$ | stands for the residue of an alcohol, $R^3OH$, commonly found in natural or synthetic pyrethroids. |

Examples of substituted or unsubstituted cycloalkyl groups named or exemplified by taking $R^1$ and $R^2$ together with the carbon to which they are attached are cyclopropyl, 2,2-dichlorocyclopropyl, cyclobutyl, cyclopentyl, and cyclohexyl. These compounds are asserted to be highly insecticidal and acaricidal and to have low toxicity to mammals and fish.

Belgian patent 902147 discloses a class of compounds having the following generic formula:

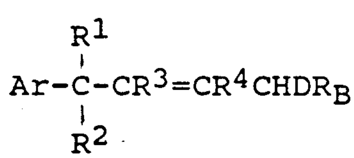

wherein

| | |
|---|---|
| Ar | represents a substituted or unsubstituted phenyl or naphthyl group; |
| $R^1$ and $R^2$ | taken together with the carbon atom to which they are attached represent a substituted or |

unsubstituted cycloalkyl group of 3-6 carbon atoms;

$R^3$ and $R^4$, which may be the same or different, are hydrogen, halogen, or $C_1$-$C_6$ alkyl;

$R_B$ represents the residue of an alcohol, $R_BCHDOH$, which provides significant insecticidal activity when esterified with 1R,cis-3-(2,2-dibromoethenyl)-2,2-dimethylcyclopropanecarboxylic acid; and

D is hydrogen or cyano.

United Kingdom patent application GB 2 085 006A discloses that insecticidal compounds of the formula

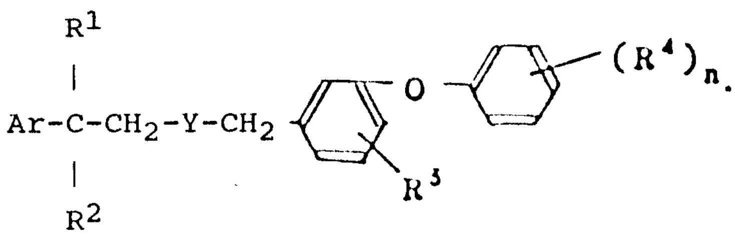

wherein

Ar is aryl,

$R^1$ is straight or branched chain alkyl of 1 - 6 carbons,

$R^2$ is hydrogen, methyl, or ethyl,

$R^3$ is halogen, methyl, or methoxyl,

$R^4$ is hydrogen, halogen, lower alkyl, or lower alkoxy, and

n is 1 or 2, with the proviso that when n is 2, the groups $R^4$ may be the same or different, and

Y is oxygen or sulfur,

are relatively low in toxicity to fishes.

International application WO 85/04651 also discloses a class of insecticides of relatively low toxicity to fishes, specifically compounds of the formula

$R_ACR_3 = CR_4CHDR_B$

wherein

$R_A$ represents a group $ArCR_1R_2$ - in which Ar represents a phenyl or naphthyl group optionally substituted by one or more halogen, alkoxy, haloalkoxy, methylenedioxy, $C_1$-$C_6$ alkyl or haloalkyl groups;

$R_1$ and $R_2$ together with the carbon to which they are attached represent a $C_3$-$C_6$ cycloalkyl group optionally substituted by one or more halogen atoms or $C_1$-$C_6$ alkyl groups.

$R_3$ and $R_4$ which may be identical or differ, represent hydrogen, halogen or $C_1$-$C_6$ alkyl groups and

$R_B$ represents the residue of an alcohol $R_B$ CHDOH in which D is hydrogen or cyano and of which the [1R, cis]2,2-dimethyl-3-(2,2-dibromovinyl) cyclopropane carboxylic ester is significantly insecticidal,

the configuration of $R_A$ and $CHDR_B$ about the double bond being mutually trans.

The compounds of this invention may be described as 2-(optionally substituted aryl)-2-cyclopropylethyl substituted-benzyl ethers and thioethers and 1-(optionally substituted aryl)-1-cyclopropyl-4-(substituted aryl)-butanes. These compounds contain an asymmetric carbon atom; the invention thus includes individual stereo-isomers as well as racemic and non-racemic mixtures of enantiomers of the instant compounds.

This invention also encompasses insecticidal compositions containing the pyrethroid ethers, thioethers, and butanes and their use for controlling insects. The compounds of this invention are effective for control of a wide variety of insects and acarids and may be expected to be useful in any situation for which pyrethroid insecticides are indicated. The compounds of this invention find particular utility in applications where there is a possibility of significant contamination of streams, rivers, and lakes by insecticidal material. Their low toxicity to fish will obviate concern about potential ecological problems associated with the use of pyrethroids in environments where such contamination is possible.

The 2-(optionally substituted aryl)-2-cyclopropylethyl substituted-benzyl ethers, thioethers, and the 1-(optionally substituted aryl)-1-cyclopropyl-4-(substituted aryl)butanes have the general formula:

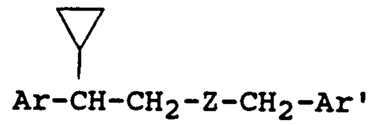

in which Ar is a thienyl or a phenyl optionally substituted with one or two groups selected from straight or branched chain ($C_{1-6}$)alkyl, halo, ($C_{1-4}$)haloalkyl, ($C_{1-4}$)alkoxy, ($C_{1-4}$)haloalkoxy, or a group having the structure -A-($CR^1R^2$)$_n$-A-, where $R^1$ and $R^2$ are independently, hydrogen, halogen, or ($C_{1-2}$)alkyl, n is 1 or 2, and each A, which may be O, S, or $CH_2$, is bonded to a carbon atom of the aromatic ring, the carbons to which the A groups are attached being adjacent to each other in the ring. Preferably Ar is phenyl and is monosubstituted at the 4-position. Halo includes fluoro, chloro, and bromo. The term alkyl includes straight and branched chain alkyl groups having 1-6 carbon atoms, preferably 1-4 carbon atoms. The terms haloalkyl and haloalkoxy include alkyl and alkoxy groups in which one or more hydrogen atoms have been replaced by fluorine, chlorine, or bromine atoms including all combinations thereof.

Illustrative of this mode of substitution are compounds in which Ar is 1,3-benzodioxolyl, 2,2-difluoro-1,3-benzodioxolyl, or 2,3-dihydro-2,2-dimethylbenzofuranyl.

Typical Ar groups include:

phenyl, fluorophenyl, chlorophenyl, bromophenyl, preferably 4-chlorophenyl;

methylphenyl, ethylphenyl, propylphenyl, isopropylphenyl, butylphenyl, isobutylphenyl, sec-butylphenyl, tert-butylphenyl, preferably methylphenyl;

methoxyphenyl, ethoxyphenyl, propoxyphenyl, isopropoxyphenyl, butoxyphenyl, isobutoxyphenyl, sec-butoxyphenyl, or tert-butoxyphenyl, preferably methoxyphenyl or ethoxyphenyl;

fluoromethylphenyl, chloromethylphenyl, trifluoromethylphenyl, difluoromethylphenyl, fluoroethylphenyl, chloroethylphenyl, preferably trifluoromethylphenyl;

difluoromethoxyphenyl, trifluoromethoxyphenyl, 2-fluoroethoxyphenyl, 1,1,2,2-tetrafluoroethoxyphenyl, 2-bromo-1-1,2,2-tetrafluoroethoxyphenyl, preferably trifluoromethoxyphenyl or difluoromethoxyphenyl;

1,3-benzodioxol-5-yl, 2,2-difluoro-1,3-benzodioxol-5-yl, naphthyl, thienyl, 2,3-dihydro-2,2-dimethylbenzofuran-5-yl, 2,2,3,3-tetrafluorobenzofuran-5-yl, and 2,3-dihydro-2,2-dimethylbenzofuran-7-yl;

Z is oxygen, sulfur, or methylene;

Ar' is 2-methyl[1,1'-biphenyl]-3-yl, 3-phenoxyphenyl, 4-fluoro-3-phenoxyphenyl, and 6-phenoxy-2-pyridyl, preferably 4-fluoro-3-phenoxyphenyl. Substitution of the phenyl, pyridyl, or phenoxy moieties with halogen or lower alkyl is within the scope of this invention.

The ether and thioether compounds of this invention are prepared by reacting an appropriate 2,2-disubstituted ethanol or thioethanol with sodium hydride, thus preparing the corresponding sodium ethoxide. The ethoxide or thioethoxide can, in turn, be reacted with an appropriately substituted benzyl halide to prepare the insecticidal ether or thioether. Example 1 describes the reaction of 2-cyclopropyl-2-(4-chlorophenyl)ethanol with sodium hydride in tetrahydrofuran and the reaction of the resulting sodium salt with (4-fluoro-3-phenoxyphenyl)methyl chloride to prepare (4-fluoro-3-phenoxyphenyl)methyl 2-cyclopropyl-2-(4-chlorophenyl)ethyl ether, Compound 16 of Table 1.

Numerous references describe the preparation of the substituted halides or preparation of the corresponding alcohols from which the halides may be prepared by conventional methods. The halides may be selected from chlorides, bromides, or iodides. Other leaving groups that may be readily displaced by a substituted ethoxide or thioethoxide may be substituted for the halogen atom of the benzyl halide. Examples of such leaving groups include, but are not limited to, methanesulfonate, trifluoromethanesulfonate, and p-toluenesulfonate.

The alcohol intermediates may be prepared from the aryl cyclopropyl ketones by conventional methods. In Example 1 the 4-chlorophenyl cyclopropyl ketone is reacted with sodium hydride and methyl triphenylphosphonium bromide to prepare 1-(4-chlorophenyl)-1-cyclopropylethene. Hydroboration of this olefin with bis(3-methyl-2-butyl)borane, followed by treatment with aqueous sodium hydroxide and hydrogen peroxide completes the synthesis of the ethanol from which the ether may be prepared as described above.

The substituted ethanol may be converted to the corresponding ethanethiol by reacting triphenylphosphine with diisopropyl azodicarboxylate and then reacting the resulting intermediate with the substituted ethanol. Quenching this reaction with thiolacetic acid produces the thiolacetate. Reduction of the thiolacetate produces the substituted thiol from which the thioether can be prepared by the same method described above for the ethers. Example 2 details the synthesis of (4-fluoro-3-phenoxy)methyl 2-cyclopropyl-2-(4-chlorophenyl)ethyl thioether, Compound 21 of Table 1 by this method.

Separation of the optical isomers can be effected by first preparing the 2,2-disubstituted acetic acid.

One method for this preparation is to react the aryl cyclopropyl ketone with the anion prepared from 2-trimethylsilyl-1,3-dithiane and n-butyllithium. The resulting 2-[(aryl)cyclopropylmethylene]-1,3-dithiane may then be reacted with mercury (II) chloride, water, and methanol, producing methyl 2-aryl-2-cyclopropylacetate. Hydrolysis of the acetate to the acid and preparation of the acid chloride may be followed by reaction with (S)-4-(1-methylethyl)-2-oxazolidinone, previously prepared by reacting (S)-2-amino-3-methyl-1-butanol with phosgene. The two diastereomers of N-(2-aryl-2-cyclopropylacetyl)-4-(1-methylethyl)-2-oxazolidinone may then be separated chromatographically. Reduction of the individual diastereomers of the oxazolidinone with lithium aluminum hydride produces the (S) or (R)-2-aryl-2-cyclopropylethanols, each substantially free of the other antipode. In Example 3 details are provided for this method of preparing the two stereoisomers of (4-fluoro-3-phenoxyphenyl)methyl 2-cyclopropyl-2-(4-chlorophenyl)ethyl ether, Compounds 17 and 18 of Table 1.

The saturated, hydrocarbon compounds of this invention are prepared by reacting a substituted-phenyl cyclopropyl ketone with vinylmagnesium bromide to prepare the corresponding 1-(substituted phenyl)-1-cyclopropyl-2-propen-1-ol. Oxidation of this unsaturated alcohol yields 3-(substituted phenyl)-3-cyclopropylpropenal. The reaction of triphenylphosphine and a substituted-benzyl bromide yields the corresponding substituted benzyltriphenylphosphonium bromide which, in turn, can be reacted with the 3-(substituted phenyl)-3-cyclopropylpropenal in the presence of n-butyllithium to yield a 1-(substituted phenyl)-1-cyclopropyl-4-(substituted phenyl)butadiene. Hydrogenation of this butadiene produces the saturated insecticidal compounds of Formula I. Example 4 details the synthesis of 1-(4-chlorophenyl)-1-cyclopropyl-4-(3-phenoxyphenyl)butane, Compound 88 of Table 1, by this method.

Alternatively, the saturated, hydrocarbon compounds may be synthesized by reacting an appropriately substituted benzaldehyde with ethoxycarbonylmethylenetriphenylphosphorane, producing the corresponding ethyl 3-(substituted aryl)acrylate. Reduction of this ester with lithium aluminum hydride yields the corresponding 3-(substituted aryl)propanol. Reaction of this alcohol with phosphorous tribromide yields the propyl bromide which, in turn, is reacted with triphenylphosphine, producing the corresponding 3-(substituted aryl)propyltriphenylphosphonium bromide. The intermediate 1-(substituted phenyl)-1-cyclopropyl-4-(substituted aryl)-1-butene is prepared by reaction of the phosphonium bromide with the appropriate substituted-phenyl cyclopropyl ketone in the presence of n-butyllithium. Catalytic hydrogenation with Raney nickel completes the synthesis. By this method 1-(4-chlorophenyl)-1-cyclopropyl-4-(3-phenoxyphenyl)butane, Compound 88 of Table 1, was synthesized as described in Example 7.

Certain substituted-phenyl cyclopropyl ketones, e.g., 4-chlorophenyl cyclopropyl ketone, are commercially available. Others can be synthesized by starting with an appropriately substituted benzoic acid which can be converted to the acid chloride by the usual methods, e.g., by reaction with oxalyl chloride. Reaction of the acid chloride with N-methoxy-N-methylamine hydrochloride yields the corresponding substituted N-methoxy-N-methylbenzamide. The desired substituted-phenyl cyclopropyl ketone is then obtained by reacting the benzamide with cyclopropylmagnesium bromide. Example 5, Steps A-C, representative of this method, provide details for the synthesis of cyclopropyl (4-trifluoromethylphenyl) ketone.

Alternatively, the substituted-phenyl cyclopropyl ketones may be prepared by reacting cyclopropanecarboxylic acid chloride with an appropriately substituted-phenyl compound in the presence of a Friedel-Crafts catalyst, e.g., aluminum chloride. In Example 6, Step A, cyclopropanecarboxylic acid chloride is reacted with ethoxybenzene in the presence of aluminum chloride, yielding cyclopropyl (4-ethoxyphenyl) ketone.

The intermediate butadienes of the formula:

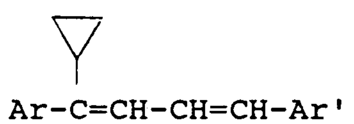

wherein Ar and Ar' are defined as above are themselves insecticidal and acaricidal. Table 2 lists these compounds.

Also, the intermediate butenes of the formula:

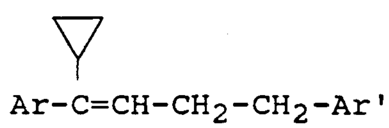

wherein Ar and Ar' are defined as above are insecticidal and acaricidal. Table 3 lists these compounds. These olefins may exist in two configurations, the E and Z isomers. In the E isomer the cyclopropyl group and the $-CH_2CH_2Ar'$ moiety are in a cis configuration in relation to the double bond and in the Z isomer these same moieties are situated in a trans configuration. In one instance an example of a Z isomer, Compound B13, was separated by rotating disk thin layer chromatography from a mixture of E and Z isomers. This enriched the residue, Compound B12, in the E isomer relative to the Z isomer. Comparisons of the insecticidal data for these compounds indicate that E isomers are significantly more active than the Z isomers.

The following examples provide additional details of the synthetic methods used to prepare the insecticidal ethers, thioethers, and hydrocarbons of this invention. Tables 1, 2, and 3 list these compounds. The compound numbers shown in each example are those assigned in these tables.

Example 1

Synthesis of (4-fluoro-3-phenoxyphenyl)methyl 2-cyclopropyl-2-(4-chlorophenyl)ethyl ether [Compound 16]

Step A Synthesis of 1-cyclopropyl-1-(4-chlorophenyl)ethene as an intermediate

Under a nitrogen atmosphere, a stirred suspension of 1.6 grams (0.063 mole) of 97% sodium hydride in 50 mL of dimethyl sulfoxide was heated at 80°C for 90 minutes. The reaction mixture was cooled to ambient temperature, and 20.8 grams (0.056 mole) of methyl triphenylphosphonium bromide was added portionwise. Upon completion of addition, an additional 20 mL of dimethylsulfoxide was added to the reaction mixture which was then stirred at ambient temperature for 30 minutes and then at 60°C for 30 minutes. The reaction mixture was cooled to ambient temperature, and 10.2 grams (0.056 mole) of cyclopropyl (4-chlorophenyl) ketone was added portionwise during a 15 minute period. Upon completion of addition, an additional 20 mL of dimethylsulfoxide was added, and the reaction mixture was stirred at ambient temperature for 18 hours. The reaction mixture was stirred with 100 mL of water which caused the precipitation of the by-product triphenylphosphine oxide. The aqueous layer and the precipitate were extracted with five 100 mL portions of hexane. The combined extracts were washed first with 80 mL of 1:1 dimethylsulfoxide:water and then with 80 mL of an aqueous, saturated sodium chloride solution. The organic layer was dried with magnesium sulfate and filtered. The filtrate was concentrated, yielding 10.9 grams of residual oil. A 1.2 gram sample from a previous run of this reaction was combined with the product of the reaction, and the 12.1 gram sample was distilled under reduced pressure, yielding 8.2 grams of 1-cyclopropyl-1-(4-chlorophenyl)ethene; b.p. 100-105°C/34 mm. The nmr and ir spectra were consistent with the proposed structure.

Step B Synthesis of 2-cyclopropyl-2-(4-chlorophenyl)ethanol as an intermediate

Under a nitrogen atmosphere, a stirred solution of 3.5 grams (0.019 mole) of 1-cyclopropyl-1-(4-chlorophenyl)ethene in 10 mL of distilled tetrahydrofuran was cooled to 0°C, and 29.5 mL (0.020 mole) of 0.68 M bis(3-methyl-2-butyl)borane in tetrahydrofuran was added via syringe during a 10 minute period. Upon completion of addition, the reaction mixture was stirred at 0°C for 1.3 hours, at ambient temperature for 2.5 hours, and at 60°C for 0.75 hour. The reaction mixture was cooled to 0°C, and 17 mL of methanol, 8.8 mL of aqueous 10% sodium hydroxide solution, and 8.0 mL of aqueous 30% hydrogen peroxide solution were sequentially added. Upon completion of addition, the reaction mixture was stirred at ambient temperature for 18 hours. The reaction mixture was heated at 60°C for 30 minutes and then was cooled, after which 30 mL of an aqueous solution saturated with potassium carbonate was added. The aqueous layer was separated and extracted with three 30 mL portions of diethyl ether. The organic materials were combined and washed with 30 mL of an aqueous, saturated potassium carbonate solution. The organic layer was dried with magnesium sulfate/potassium carbonate and filtered. The filtrate was concentrated under reduced pressure, yielding 3.8 grams of 2-cyclopropyl-2-(4-chlorophenyl)ethanol. The nmr and ire spectra were consistent with the proposed structure.

Step C Synthesis of (4-fluoro-3-phenoxyphenyl)methyl 2-cyclopropyl-2-(4-chlorophenyl)ethyl ether

A stirred suspension of 0.1 gram (0.0044 mole) of sodium hydride in 5 mL of tetrahydrofuran was cooled to 0°C, and a solution of 0.8 gram (0.0041 mole) of 2-cyclopropyl-2-(4-chlorophenyl)ethanol in 2.5 mL of tetrahydrofuran was added via syringe during a two minute period. Upon completion of addition, the reaction mixture was allowed to warm to ambient temperature where it stirred for 30 minutes and then was

heated to 55°C where it stirred for 1.5 hours. The reaction mixture was cooled to ambient temperature, and a solution of 1.0 gram (0.0043 mole) of (4-fluoro-3-phenoxyphenyl)methyl chloride in 2.5 mL of tetrahydrofuran was added via syringe. Upon completion of addition, the reaction mixture stirred at ambient temperature for 20 hours and then was warmed to 60°C where it stirred for 30 minutes. The reaction mixture was cooled, and 15 mL of water was added. The aqueous layer was removed and extracted with three 25 mL portions of hexanes. The organic materials were combined and dried with magnesium sulfate. The mixture was filtered, and the filtrate was concentrated under reduced pressure to a residual oil. The oil was purified by rotating disk thin layer chromatography using 5-10% ethyl acetate in hexanes for elution. The appropriate fractions were combined and concentrated under reduced pressure, yielding 0.65 gram of (4-fluoro-3-phenoxyphenyl)methyl 2-cyclopropyl-2-(4-chlorophenyl)ethyl ether. The nmr and the ir spectra were consistent with the proposed structure.

Example 2

Synthesis of (4-fluoro-3-phenoxyphenyl)methyl 2-cyclopropyl-2-(4-chlorophenyl)ethyl thioether [Compound 21]

Step A Synthesis of 2-cyclopropyl-2-(4-chlorophenyl)ethyl thiolacetate as an intermediate

A stirred solution of 11.7 grams (0.045 mole) of triphenylphosphine in 75 mL of dry tetrahydrofuran was cooled to 0°C, and 9.0 grams (0.045 mole) of diisopropyl azodicarboxylate was added dropwise. Upon completion of addition, the reaction mixture was allowed to warm to ambient temperature where it stirred for 30 minutes. Successively, 4.4 grams (0.022 mole) of 2-cyclopropyl-2-(4-chlorophenyl)ethanol (prepared in Example 1, Step B) and 3.4 grams (0.045 mole) of thiolacetic acid were then added. The exothermic reaction caused the reaction mixture temperature to rise to 39°C. After cooling to ambient temperature the reaction mixture was stirred for 16 hours. The reaction mixture was then concentrated under reduced pressure to a residual oil. The oil was subjected to chromatography on silica gel using methylene chloride:heptane (1:4) as eluant. The appropriate fractions were combined and concentrated under reduced prssure, yielding 4.6 grams of 2-cyclopropyl-2-(4-chlorophenyl)ethyl thiolacetate as an oil. The nmr and the ir spectra were consistent with the proposed structure.

Step B Synthesis of 2-cyclopropyl-2-(4-chlorophenyl)ethanethiol as an intermediate

Under a nitrogen atmosphere a mixture of 1.2 grams (0.032 mole) of lithium aluminum hydride in dry tetrahydrofuran was stirred, and a solution of 4.1 grams (0.016 mole) of 2-cyclopropyl-2-(4-chlorophenyl)-ethyl thiolacetate in 3 mL of tetrahydrofuran was added dropwise. Upon completion of addition, the reaction mixture was stirred at ambient temperature for 16 hours. Water was then carefully added dropwise to destroy excess lithium aluminum hydride. After the hydride was destroyed, 50 mL of additional water was added. The reaction mixture was extracted with several portions of diethyl ether. The combined extracts were dried with magnesium sulfate and filtered. The filtrate was concentrated under reduced pressure, yielding 3.4 grams of 2-cyclopropyl-2-(4-chlorophenyl)ethanethiol. The nmr and the ir spectra were consistent with the proposed structure.

Procedures analogous to Steps A and B are reported in Tetrahedron Letters, Vol. 22, No. 33, p 3119-3122, 1981.

Step C Synthesis of (4-fluoro-3-phenoxyphenyl)methyl 2-cyclopropyl-2-(4-chlorophenyl)ethyl thioether

This compound was prepared in a manner analogous to that of Example 1, Step C, using 1.0 gram (0.0046 mole) of 2-cyclopropyl-2-(4-chlorophenyl)ethanethiol, 0.97 gram (0.0041 mole) of (4-fluoro-3-phenoxyphenyl)methyl chloride, and 0.22 gram (0.0055 mole) of sodium hydride in 12 mL of dry tetrahydrofuran. The yield of (4-fluoro-3-phenoxyphenyl)methyl 2-cyclopropyl-2-(4-chlorophenyl)ethyl thioether was 1.2 grams as an oil. The nmr and the ir spectra were consistent with the proposed structure.

Example 3

Synthesis of the stereoisomers (A) and (B) of (4-fluoro-3-phenoxyphenyl)methyl 2-cyclopropyl-2-(4-chlorophenyl)ethyl ether [Compounds 17 and 18, respectively]

Step A Synthesis of 2-[(4-chlorophenyl)cyclopropylmethylene]-1,3-dithiane as an intermediate

A solution of 16.0 grams (0.083 mole) of 2-trimethylsilyl-1,3-dithiane in 80 mL of tetrahydrofuran was cooled to 0°C, and 39 mL (0.083 mole) of n-butyllithium (2.1 M in hexane) was added. The reaction mixture was stirred for 15 minutes, and 15.0 grams (0.083 mole) of cyclopropyl (4-chlorophenyl) ketone in 40 mL of tetrahydrofuran was added via syringe during a five minute period. Upon completion of addition, the reaction mixture was stirred at 0°C for 15 minutes and then was allowed to warm for 30 minutes. The reaction mixture was stirred with 100 mL of an aqueous solution saturated with sodium chloride, and then the two phases were separated. The aqueous phase was extracted with one portion of diethyl ether. The ether extract was combined with the organic phase, and this mixture was dried with magnesium sulfate and filtered through a pad of silica gel. The filtrate was concentrated under reduced pressure, yielding 24.0 grams of 2-[(4-chlorophenyl)cyclopropylmethylene]-1,3-dithiane as a solid, m.p. 91-95°C. The nmr spectrum was consistent with the proposed structure.

Step B Synthesis of methyl 2-cyclopropyl-2-(4-chlorophenyl)acetate as an intermediate

A mixture of 10.0 grams (0.036 mole) of 2-[(4-chlorophenyl)cyclopropylmethylene]-1,3-dithiane, 24.0 grams (0.086 mole) of mercury (II) chloride, and 5 mL of water in 50 mL of methanol was stirred at ambient temperature for 18 hours. The reaction mixture was heated at reflux for one hour, cooled, and then was diluted with diethyl ether. The mixture was filtered through diatomaceous earth and then was dried with magnesium sulfate. The mixture was filtered through silica gel, and the filtrate was concentrated under reduced pressure, yielding 5.9 grams of methyl 2-cyclopropyl-2-(4-chlorophenyl)acetate as an oil. The nmr spectrum was consistent with the proposed structure. The reaction was run again to produce an additional 7.1 grams of the acetate.

Step C Synthesis of 2-cyclopropyl-2-(4-chlorophenyl)acetic acid as an intermediate

A mixture of 13.0 grams (0.058 mole) of methyl 2-cyclopropyl-2-(4-chlorophenyl)acetate and 5.0 grams of an aqueous, 50% sodium hydroxide solution in 50 mL of methanol was stirred at ambient temperature for 18 hours. The reaction mixture was diluted with 150 mL of water, and the solution was decanted from a solid residue. The liquid portion was washed with three portions of diethyl ether. The combined ether washes were, in turn, washed with an aqueous, dilute sodium hydroxide solution. The combined aqueous layers were made acidic by the slow addition of aqueous, 10% hydrochloric acid. The acidified mixture was extracted with five portions of methylene chloride. The combined extracts were dried with sodium sulfate and filtered. The filtrate was concentrated under reduced pressure, yielding 7.4 grams of 2-cyclopropyl-2-(4-chlorophenyl)acetic acid as a solid, m.p. 95-96°C

Step D Synthesis of (S)-4-(1-methylethyl)-2-oxazolidinone as an intermediate

A mixture of 9.4 grams (0.091 mole) of (S)-2-amino-3-methyl-1-butanol, 36 grams (0.0546 mole) of 85% potassium hydroxide, 175 mL of toluene, and 240 mL of water was stirred rapidly as 140 mL (0.273 mole) of a toluene solution containing 20% phosgene was added dropwise during a 15 minute period. Upon completion of addition, the resulting hot solution was stirred an additional 30 minutes. The reaction mixture was cooled, and the organic and aqueous layers were separated. The organic layer was washed with water and dried with magnesium sulfate. The mixture was filtered, and the filtrate was concentrated under reduced pressure, yielding 15.0 grams of (S)-4-(1-methylethyl)-2-oxazolidinone as a solid. Recrystallization from cyclohexane yielded purer compound, m.p. 71.5-72.5°C.

Step E Synthesis of (S)-N-[2-(4-chlorophenyl)-2-cyclopropylacetyl]-4-(1-methylethyl)-2-oxazolidinone and separation of its diastereomers (A) and (B) for use as intermediates

A solution of 3.3 grams (0.016 mole) of 2-cyclopropyl-2-(4-chlorophenyl)acetic acid (prepared in Step C), 1.36 mL (0.016 mole) of oxalyl chloride, and two drops of N,N-dimethylformamide in 70 mL of diethyl ether was stirred at 0°C for one hour. The reaction mixture was then allowed to warm to ambient temperature where it stirred for one hour.

In a separate reaction vessel a stirred solution of 2.0 grams (0.016 mole) of (S)-4-(1-methylethyl)-2-oxazolidinone in 50 mL of tetrahydrofuran was cooled to -78°C, and 6.25 mL (0.016 mole) of n-butyllithium (2.5 molar in hexane) was added dropwise. Upon completion of addition, the reaction mixture was stirred for

30 minutes, and then the 2-cyclopropyl-2-(4-chlorophenyl)acetyl chloride, prepared above, was added dropwise during several minutes. Upon completion of addition, the reaction mixture was stirred for an additional 30 minutes and then was poured into water. The organic layer was separated and washed with one portion of aqueous sodium bicarbonate. The organic layer was dried with magnesium sulfate and filtered. The filtrate was concentrated under reduced pressure to a residual oil. The oil was subjected to chromatography on silica gel using hexane:diethyl ether (3:1) as eluant. The appropriate fractions were combined and concentrated under reduced pressure, yielding 0.85 gram of diastereomer (A) and 0.8 gram of diastereomer (B) of (S)-N-[2-(4-chlorophenyl)-2-cyclopropylacetyl]-4-(1-methylethyl)-2-oxazolidinone. Upon standing, diastereomer (A) crystallized to a solid, m.p. 61-64°C.

Step F Synthesis of stereoisomer (A) of 2-cyclopropyl-2-(4-chlorophenyl)ethanol as an intermediate

A stirred suspension of 0.31 gram (0.008 mole) of lithium aluminum hydride in 5 mL of tetrahydrofuran was cooled to 0°C, and 0.85 gram (0.0026 mole) of diastereomer (A) of (S)-N-[2-(4-chlorophenyl)-2-cyclopropylacetyl]-4-(1-methylethyl)-2-oxazolidinone was added. Upon completion of addition, the reaction mixture was stirred for 45 minutes, and then 15 mL of hexane was added to the reaction mixture. This was followed by the careful addition of 0.3 mL of water, 0.3 mL of aqueous 15% sodium hydroxide, and 0.9 mL of water. The reaction mixture was stirred with magnesium sulfate and filtered through a pad of silica gel. The filtrate was concentrated under reduced pressure to a residual oil. The oil was subjected to chromatography on silica gel using diethyl ether:hexane (1:1) as eluant. The appropriate fractions were combined and concentrated under reduced pressure, yielding 0.4 gram of stereoisomer (A) of 2-cyclopropyl-2-(4-chlorophenyl)ethanol as an oil.

Step G Synthesis of stereoisomer (A) of (4-fluoro-3-phenoxyphenyl)methyl 2-cyclopropyl-2-(4-chlorophenyl)-ethyl ether

Under a nitrogen atmosphere a suspension of 0.06 gram (0.0024 mole) of 97% sodium hydride in 2.2 mL of dimethylformamide was stirred, and a solution of 0.40 gram (0.002 mole) of stereoisomer (A) of 2-cyclopropyl-2-(4-chlorophenyl)ethyl ethanol in 1.0 mL of dimethylformamide was slowly added. Upon completion of addition, the reaction mixture gas stirred for 1.5 hours, and then a solution of 0.46 gram (0.0019 mole) of (4-fluoro-3-phenoxyphenyl)methyl chloride in 1.0 mL of dimethylformamide was added. Upon completion of addition, the reaction mixture was stirred for one hour, and then 2-3 mL of water was added. The mixture was poured into 75 mL of aqueous, 10% hydrochloric acid and then was extracted with two 50 mL portions of hexane. The combined hexane layers were washed with 25 mL of a saturated, aqueous solution of sodium chloride. The organic layer was dried with sodium sulfate and filtered. The filtrate was concentrated under reduced pressure to a residual oil. The oil was subjected to rotating disk thin layer chromatography on silica gel using diethyl ether: hexane (19:1) as eluant. The appropriate fractions were combined and concentrated under reduced pressure, yielding 0.55 gram of stereoisomer (A) of (4-fluoro-3-phenoxyphenyl)methyl 2-cyclopropyl-2-(4-chlorophenyl)ethyl ether as an oil. The nmr spectrum was consistent with the proposed structure. $[\alpha]_D^{25} = (+)22.19°$

Step H Synthesis of stereoisomer (B) of 2-cyclopropyl-2-(4-chlorophenyl)ethanol as an intermediate

This compound was prepared in a manner analogous to that of Step F, using 0.80 gram (0.0025 mole) of diastereomer (B) of (S)-N-[2-(4-chlorophenyl)-2-cyclopropylacetyl]-4-(1-methylethyl)-2-oxazolidinone (prepared in Example 3, Step E) and 0.30 gram (0.008 mole) of lithium aluminum hydride in 15 mL of tetrahydrofuran. The yield of stereoisomer (B) of 2-cyclopropyl-2-(4-chlorophenyl)ethanol was 0.45 gram as an oil.

Step I Synthesis of stereoisomer (B) of (4-fluoro-3-phenoxyphenyl)methyl 2-cyclopropyl-2-(4-chlorophenyl)

This compound was prepared in a manner analogous to that of Step G, using 0.40 gram (0.0020 mole) of stereoisomer (B) of 2-cyclopropyl-2-(4-chlorophenyl)ethanol (prepared in Step H), 0.46 gram (0.0019 mole) of (4-fluoro-3-phenoxyphenyl)methyl chloride, and 0.06 gram (0.0024 mole) of sodium hydride in 4.2 mL of dimethylformamide. The yield of stereoisomer (B) of (4-fluoro-3-phenoxyphenyl)methyl 2-cyclopropyl-2-(4-chlorophenyl)ethyl ether was 0.56 gram as an oil. The nmr spectrum was consistent with the proposed structure. $[\alpha]_D^{25} = (-)20.64°$

Example 4

Synthesis of 1-(4-chlorophenyl)-1-cyclopropyl-4-(3-phenoxyphenyl)butane [Compound 88]

Step A Synthesis of 1-(4-chlorophenyl)-1-cyclopropyl-2-propen-1-ol as an intermediate

A 1.0 M solution of vinylmagnesium bromide in tetrahydrofuran (110 mL, 0.11 mole) was stirred, and a solution of 18.1 grams (0.1 mole) of commercially available 4-chlorophenyl cyclopropyl ketone in 50 mL of dry tetrahydrofuran was added dropwise during a one hour period. The exothermic reaction caused the reaction mixture to warm to 45°C. Upon completion of addition, the reaction mixture was stirred for two hours as it cooled to ambient temperature. The reaction was quenched with the addition of 50 mL of a saturated, aqueous solution of ammonium chloride. The mixture was extracted with two 50 mL portions of diethyl ether. The combined extracts were dried with potassium carbonate and filtered. The filtrate was concentrated under reduced pressure, yielding 20.0 grams of 1-(4-chlorophenyl)-1-cyclopropyl-2-propen-1-ol.

Step B Synthesis of 3-(4-chlorophenyl)-3-cyclopropylpropenal as an intermediate

To a stirred solution of 40.3 grams (0.192 mole) of pyridinium chlorochromate in 210 mL of methylene chloride was added a solution of 20.0 grams (0.096 mole) of 1-(4-chlorophenyl)-1-cyclopropyl-2-propen-1-ol in 25 mL of methylene chloride in one portion. Upon completion of addition, the reaction mixture was stirred for two hours. A supernatent layer was decanted from a residue, and the residue was extracted with diethyl ether. The supernatent layer was combined with the ether extracts, and the combination was washed with two 100 mL portions of an aqueous 5% sodium hydroxide solution, 100 mL of an aqueous 5% hydrochloric acid solution, and then with 50 mL of an aqueous solution saturated with sodium bicarbonate. The organic layer was dried with sodium sulfate and filtered. The filtrate was concentrated under reduced pressure to a residue. The residue was subjected to column chromatography on silica gel. Elution was accomplished using 5% diethyl ether in hexane. The appropriate fractions were combined and concentrated under reduced pressure, yielding 6.8 grams of 3-(4-chlorophenyl)-3-cyclopropylpropenal.

Step C Synthesis of 3-phenoxyphenylmethyltriphenylphosphonium chloride as an intermediate

A stirred solution of 5.0 grams (0.0228 mole) of 3-phenoxyphenylmethyl chloride and 5.6 grams (0.0217 mole) of triphenyl phosphine in 50 mL of dry toluene was heated at reflux for 8 hours. The reaction mixture was cooled and filtered to collect a solid. The solid was washed with pentane and dried, yielding 4.6 grams of 3-phenoxyphenylmethyltriphenylphosphonium chloride. The nmr spectrum was consistent with the proposed structure.

Step D Synthesis of 1-(4-chlorophenyl)-1-cyclopropyl-4-(3-phenoxyphenyl)-1,3-butadiene (Compound A5) as an intermediate

A stirred solution of 4.4 mL (0.011 mole) of n-butyllithium (2.5 molar in hexane) in 100 mL of dry tetrahydrofuran was cooled to -78°C, and 4.6 grams (0.01 mole) of 3-phenoxyphenylmethyltriphenylphosphonium chloride was quickly added. Upon completion of addition, the reaction mixture was stirred at -78°C for one hour and then was allowed to warm to -20°C where it stirred for one hour. The reaction mixture was cooled to -78°C, and 2.1 grams (0.01 mole) of 3-(4-chlorophenyl)-3-cyclopropylpropenal (prepared in Step B) in 10 mL of tetrahydrofuran was added during a 15 minute period. Upon completion of addition, the reaction mixture was allowed to warm to ambient temperature where it stirred for two hours. The reaction was quenched with the addition of 15 mL of aqueous 10% hydrochloric acid solution. The mixture was extracted with diethyl ether. The combined extracts were dried with sodium sulfate and filtered. The filtrate was concentrated under reduced pressure to a residue. The residue was subjected to column chromatography on silica gel. Elution was accomplished using 5% diethyl ether in hexane. The appropriate fractions were combined and concentrated under reduced pressure, yielding 3.2 grams of 1-(4-chlorophenyl)-1-cyclopropyl-4-(3-phenoxyphenyl)-1,3-butadiene.

Step E Synthesis of 1-(4-chlorophenyl)-1-cyclopropyl-4-(3-phenoxyphenyl)butane (Compound 88)

A mixture of 2.3 grams (0.006 mole) of 1-(4-chlorophenyl)-1-cyclopropyl-4-(3-phenoxyphenyl)-1,3-butadiene, 2.3 grams (0.002 mole) of 10% palladium on carbon, 0.25 gram (0.0002 mole) of tris-(triphenylphosphine)rhodium (I) chloride, and 25 mL of benzene in 100 mL of ethanol was hydrogenated at 40°C using a Parr hydrogenator. Upon completion of the uptake of the theoretical amount of hydrogen (two hours), the reaction mixture was cooled and filtered. The filtrate was concentrated under reduced pressure to a residue. The residue was taken up in hexane and filtered. The filtrate was dried with sodium sulfate and filtered again. The filtrate was concentrated under reduced pressure to a residue. The residue was subjected to rotating disk thin layer chromatography. Elution was accomplished using 20% toluene in hexane. The appropriate fractions were combined and concentrated under reduced pressure, yielding 0.52 gram of 1-(4-chlorophenyl)-1-cyclopropyl-4-(3-phenoxyphenyl)butane as an oil. The nmr spectrum was consistent with the proposed structure.

Example 5

Synthesis of 1-cyclopropyl-1-(4-trifluoromethylphenyl)-4-(4-fluoro-3-phenoxyphenyl)butane [Compound 99]

Step A Synthesis of 4-trifluoromethylbenzoyl chloride as an intermediate

A stirred solution of 20.0 grams (0.105 mole) of 4-trifluoromethylbenzoic acid and four drops of dimethylformamide in 300 mL of methylene chloride was cooled to 0-10°C, and 14.7 grams (0.116 mole) of oxalyl chloride was added. Upon completion of addition, the reaction mixture was allowed to warm to ambient temperature where it stirred for 18 hours. The reaction mixture was then concentrated under reduced pressure, yielding 21.9 grams of 4-trifluoromethylbenzoyl chloride as a semi-solid. The reaction was repeated.

Step B Synthesis of N-methoxy-N-methyl-4-trifluoromethylbenzamide as an intermediate

To a stirred suspension of 19.9 grams (0.204 mole) of N-methoxy-N-methylamine hydrochloride in 500 mL of methylene chloride was added 39.3 grams (0.388 mole) of triethylamine. Upon completion of addition, the reaction mixture was stirred for ten minutes, and a solution of 38.4 grams (0.185 mole) of 4-trifluoromethylbenzoyl chloride in 25 mL of methylene chloride was added dropwise. Upon completion of addition, the reaction mixture was stirred at ambient temperature for 18 hours. The reaction mixture was then stirred vigorously with 300 mL of water. The aqueous layer was separated from the organic layer and washed with three portions of methylene chloride. The washes were combined with the organic layer, and the combination was dried with magnesium sulfate. The mixture was filtered, and the filtrate was concentrated under reduced pressure, yielding 42.5 grams of N-methoxy-N-methyl-4-trifluoromethylbenzamide as an oil. The nmr spectrum was consistent with the proposed structure.

Step C Synthesis of cyclopropyl (4-trifluoromethylphenyl) ketone as an intermediate

Under a nitrogen atmosphere a vigorously stirred solution of 42.5 grams (0.182 mole) of N-methoxy-N-methyl-4-trifluoromethylbenzamide in 250 mL of dry tetrahydrofuran was cooled to 0-10°C, and 41.7 grams (0.0287 mole) of freshly prepared cyclopropylmagnesium bromide in 170 mL of tetrahydrofuran was added rapidly dropwise. Upon completion of addition, the reaction mixture was allowed to warm to ambient temperature where it stirred for 60 hours. The reaction mixture was then concentrated under reduced pressure to a residue. The residue was taken up in water and extracted with four portions of methylene chloride. The combined extracts were dried with magnesium sulfate and filtered. The filtrate was passed through a pad of silica gel and was concentrated under reduced pressure yielding, 34.6 grams of cyclopropyl (4-trifluoromethylphenyl) ketone. The nmr spectrum was consistent with the proposed structure.

Step D Synthesis of 1-cyclopropyl-1-(4-trifluoromethylphenyl)-2-propen-1-ol as an intermediate

This compound was prepared in a manner analogous to that of Example 4, Step A, using 10.0 grams (0.05 mole) of cyclopropyl (4-trifluoromethylphenyl) ketone and 50 mL (0.05 mole) of vinylmagnesium bromide (1.0 M in tetrahydrofuran) and 25 mL of tetrahydrofuran. The yield of 1-cyclopropyl-1-(4-trifluoromethylphenyl)-2-propen-1-ol was 11.6 grams. The nmr spectrum was consistent with the proposed structure.

Step E Synthesis of 3-cyclopropyl-3-(4-trifluoromethylphenyl)propenal as an intermediate

This compound was prepared in a manner analogous to that of Example 4, Step B, using 11.1 grams (0.046 mole) of 1-cyclopropyl-1-(4-trifluoromethylphenyl)-2-propen-1-ol and 19.7 grams (0.091 mole) of pyridinium chlorochromate in 100 mL of methylene chloride. The yield of 3-cyclopropyl-3-(4-trifluoromethylphenyl)propenal was 5.5 grams

Step F Synthesis of 4-fluoro-3-phenoxyphenylmethanol as an intermediate

To a stirred suspension of 1.4 grams (0.0375 mole) of lithium aluminum hydride in 50 mL of anhydrous diethyl ether was added dropwise during a one hour period a solution of 21.6 grams (0.1 mole) of 4-fluoro-3-phenoxybenzaldehyde in 50 mL of anhydrous diethyl ether. Upon completion of addition, the reaction mixture was heated at reflux for 1.0 hour. The reaction mixture was cooled to 15°C, and 1.4 mL of water was cautiously added dropwise. Upon completion of addition, the reaction mixture was again cooled to 15°C, and 1.4 mL of an aqueous, 15% sodium hydroxide solution was added dropwise, followed by an additional 4.2 mL of water. The mixture was filtered through diatomaceous earth, and the filtrate was concentrated under reduced pressure, yielding 19.5 grams of 4-fluoro-3-phenoxyphenylmethanol as an oil.

Step G Synthesis of 4-fluoro-3-phenoxyphenylmethyl chloride as an intermediate

To a stirred solution of 12.6 grams (0.106 mole) of thionyl chloride and a catalytic amount of pyridine in 25 mL of toluene was added dropwise during a 45 minute period a solution of 19.5 grams (0.88 mole) of 4-fluoro-3-phenoxyphenylmethanol(prepared in Step F) in 30 mL of toluene. The reaction mixture temperature was maintained at 25-35°C throughout the addition. Upon completion of addition, the reaction mixture was warmed to 45°C where it stirred for one hour. The reaction mixture was cooled and then was concentrated under reduced pressure, yielding 23.5 grams of semi-solid. The semi-solid was combined with 114.2 grams of identical semi-solid obtained from a large run of the present reaction. The 136.6 grams of semi-solid was distilled under reduced pressure. The appropriate fractions were combined, yielding 100.3 grams of 4-fluoro-3-phenoxyphenylmethyl chloride, b.p. 98-105°C/0.03-0.13 mm Hg.

Step H Synthesis of (4-fluoro-3-phenoxyphenyl)methyltriphenylphosphonium chloride as an intermediate

This compound was prepared in a manner analogous to that of Example 4, Step C, using 11.8 grams (0.05 mole) of 4-fluoro-3-phenoxyphenylmethyl chloride and 13.1 grams (0.05 mole) of triphenylphosphine in 100 mL of tetrahydrofuran. The yield of (4-fluoro-3-phenoxyphenyl)methyltriphenylphosphonium chloride was 15.0 grams.

Step I Synthesis of 1-cyclopropyl-1-(4-trifluoromethylphenyl)-4-(4-fluoro-3-phenoxyphenyl)-1,3-butadiene (Compound A13) as an intermediate

This compound was prepared in a manner analogous to that of Example 4, Step D, using 1.7 grams (0.0069 mole) of 3-cyclopropyl-3-(4-trifluoromethylphenyl)propenal (prepared in Step E of the present example), 3.4 grams (0.0069 mole) of (4-fluoro-3-phenoxyphenyl)methyltriphenylphosphonium chloride (prepared in Step H of the present example), and 2.8 mL (0.0069 mole) of n-butyllithium (2.5 molar in hexane) in 69 mL of dry tetrahydrofuran. The yield of 1-cyclopropyl-1-(4-trifluoromethylphenyl)-4-(4-fluoro-3-phenoxyphenyl)-1,3-butadiene was 1.8 grams. The nmr spectrum was consistent with the proposed structure.

Step J Synthesis of 1-cyclopropyl-1-(4-trifluoromethylphenyl)-4-(4-fluoro-3-phenoxyphenyl)butane (Compound 99)

This compound was prepared in a manner analogous to that of Example 4, Step E, by the hydrogenation of 0.98 gram (0.0023 mole) of 1-cyclopropyl-1-(4-trifluoromethylphenyl)-4-(4-fluoro-3-phenoxyphenyl)-1,3-butadiene in the presence of 0.2 gram (0.00023 mole) of Raney nickel in 50 mL of ethanol. The yield of 1-cyclopropyl-1-(4-trifluoromethylphenyl)-4-(4-fluoro-3-phenoxyphenyl)butane was 0.65 gram as an oil. The nmr spectrum was consistent with the proposed structure.

Example 6

Synthesis of 1-cyclopropyl-1-(4-ethoxyphenyl)-4-(3-phenoxyphenyl)butane [Compound 104]

Step A Synthesis of cyclopropyl (4-ethoxyphenyl) ketone as an intermediate

Under an argon atmosphere a stirred suspension of 36.7 grams (0.275 mole) of aluminum chloride in 225 mL of carbon disulfide was cooled to 0°C, and 22.7 mL (0.25 mole) of cyclopropanecarboxylic acid chloride was added dropwise during a 15 minute period. During the addition and for 30 minutes after its completion the reaction mixture temperature was maintained at 0-15°C. Then 34.8 mL of ethoxybenzene was added dropwise during a one hour period. The reaction mixture temperature was maintained at 5-10°C during this addition. Upon completion of addition, the reaction mixture was allowed to warm to ambient temperature as it stirred for one hour. Petroleum ether, 250 mL, was added to the reaction mixture, and the suspension was stirred for ten minutes. The solid was collected by filtration and washed with petroleum ether. The solid was returned to the reaction vessel and, with stirring, was cooled to 0-10°C while 50 mL of water was added dropwise during a 30 minute period. Upon completion of addition, the mixture was stirred until the evolution of hydrogen chloride ceased. An additional 250 mL of water was then added, and the mixture was stirred at ambient temperature for 30 minutes. It was then warmed to 80°C where it stirred for an additional 30 minutes. The mixture was cooled, and a solid was collected by filtration. The solid was dissolved in methylene chloride, and the solution was dried with sodium sulfate. The mixture was filtered, and the filtrate was concentrated under reduced pressure to a residual solid. The solid was recrystallized from heptane, yielding, in two crops, 44.0 grams of cyclopropyl (4-ethoxyphenyl) ketone, m.p. 67-70°C. The nmr spectrum was consistent with the proposed structure.

Step B Synthesis of 1-cyclopropyl-1-(4-ethoxyphenyl)-2-propen-1-ol as an intermediate

This compound was prepared in a manner analogous to that of Example 4, Step A, using 5.7 grams (0.03 mole) of cyclopropyl (4-ethoxyphenyl) ketone and 33 mL (0.033 mole) of vinylmagnesium bromide (1.0 M in tetrahydrofuran) in 30 mL of dry tetrahydrofuran. The yield of 1-cyclopropyl-1-(4-ethoxyphenyl)-2-propen-1-ol was 6.5 grams as an oil.

Step C Synthesis of 3-cyclopropyl-3-(4-ethoxyphenyl)propenal as an intermediate

This compound was prepared in a manner analogous to that of Example 4, Step B, using 6.5 grams (0.029 mole) of 1-cyclopropyl-1-(4-ethoxyphenyl)-2-propen-1-ol and 15.3 grams (0.029 mole) of pyridinium dichromate in 40 mL of methylene chloride. The yield of 3-cyclopropyl-3-(4-ethoxyphenyl)propenal was 4.2 grams as an oil.

Step D Synthesis of 1-cyclopropyl-1-(4-ethoxyphenyl)-4-(3-phenoxyphenyl)-1,3-butadiene (Compound A15) as an intermediate

This compound was prepared in a manner analogous to that of Example 4, Step D, using 4.2 grams (0.019 mole) of 3-cyclopropyl-3-(4-ethoxyphenyl)propenal, 9.1 grams (0.019 mole) of 3-phenoxyphenylmethyltriphenylphosphonium bromide (prepared as in Example 4, Step H), and 7.5 mL (0.019 mole) of n-butyllithium (2.5 M in tetrahydrofuran) in 100 mL of dry tetrahydrofuran. The yield of 1-cyclopropyl-1-(4-ethoxyphenyl)-4-(3-phenoxyphenyl)-1,3-butadiene was 2.5 grams.

Step E Synthesis of 1-cyclopropyl-1-(4-ethoxyphenyl)-4-(3-phenoxyphenyl)butane (Compound 104)

This compound was prepared in a manner analogous to that of Example 4, Step E, by the hydrogenation of 1.5 grams (0.0039 mole) of 1-cyclopropyl-1-(4-ethoxyphenyl)-4-(3-phenoxyphenyl)-1,3-butadiene in the presence of 0.34 gram of Raney nickel in 70 mL of ethanol. The yield of 1-cyclopropyl-1-(4-ethoxyphenyl)-4-(3-phenoxyphenyl)butane was 1.2 grams as an oil. The nmr spectrum was consistent with the proposed structure.

Example 7

Synthesis of 1-(4-chlorophenyl)-1-cyclopropyl-4-(3-phenoxyphenyl)butane [Compound 88]

Step A Synthesis of ethyl 3-(3-phenoxyphenyl)acrylate

To a stirred solution of 23.4 g (0.188 mole) of 3-phenoxybenzaldehyde in 175 mL of 1,4-dioxane was

added 45.2 grams (0.130 mole) of ethoxycarbonylmethylenetriphenylphosphorane in one portion. The reaction was allowed to stir at ambient temperature overnight. The solvent was evaporated under reduced pressure, leaving a residue which was dissolved in ethyl acetate. Approximately 30 grams of silica gel was mixed with this solution. This solvent was evaporated under reduced pressure, and the silica gel was placed in a sintered glass filter. The silica gel was eluted with 1000 mL of heptane/ethyl acetate (3:1). The solvent was evaporated under reduced pressure, leaving an oil. This oil was dissolved in 150 mL of heptane/ethyl acetate (9:1), treated with 15 grams of silica gel, and filtered. The filtrate was evaporated under reduced pressure, leaving 26.7 grams of ethyl 3-(3-phenoxyphenyl)acrylate as an oil. The nmr spectrum was consistent with the proposed structure.

Step B Synthesis of 3-(3-phenoxyphenyl)propanol

To a stirred mixture of 7.4 grams (0.196 mole) of lithium aluminum hydride in 300 mL of dry diethyl ether under a nitrogen atmosphere was added 26.2 grams (0.098 mole) of ethyl 3-(3-phenoxyphenyl)-acrylate in 300 mL of dry diethyl ether. The addition required 90 minutes to complete, and the reaction mixture was stirred overnight at ambient temperature. It was then cooled in an ice/water bath, and sequentially 14 mL of water, 14 mL of a 15% aqueous solution of sodium hydroxide, and 42 mL of water were all added dropwise. This mixture was filtered, and the filtrate was dried over anhydrous sodium sulfate. After being filtered, the solvent was evaporated under reduced pressure, leaving 21.9 grams of 3-(3-phenoxyphenyl)propanol as an oil. The nmr spectrum was consistent with the proposed structure.

Step C Synthesis of 3-(3-phenoxyphenyl)propyl bromide

To a mixture of 21.0 grams (0.092 mole) of 3-(3-phenoxyphenyl)propanol and 1 mL of pyridine which had been cooled to 0°C was added dropwise during a 20 minute period 8.27 grams (0.031 mole) of phosphorus tribromide. This mixture was stirred at 0°C for 90 minutes and then at ambient temperature overnight. The reaction mixture was then diluted with 200 mL of diethyl ether, and the solution was washed successively twice with 50 mL of water, four times with 25 mL of a saturated, aqueous solution of sodium bicarbonate, once with 50 mL of water, and once with an aqueous solution of sodium chloride. After being dried over anhydrous sodium sulfate and filtered, the solvent was evaporated under reduced pressure, leaving 18.9 grams of 3-(3-phenoxyphenyl)propyl bromide as an oil. The nmr spectrum was consistent with the proposed structure.

Step D Synthesis of 3-(3-phenoxyphenyl)propyltriphenylphosphoniumbromide

Under nitrogen a mixture of 2.9 grams (0.01 mole) of 3-(3-phenoxyphenyl)propyl bromide and 2.9 grams (0.01 mole) of triphenylphosphine in 25 mL of acetonitrile was heated at reflux overnight. The solvent was evaporated under reduced pressure. Toluene was added to the residue, and this mixture was heated at reflux for 90 minutes during which a solid formed. Filtration yielded 4.2 grams of 3-(3-phenoxyphenyl)-propyltriphenylphosphonium bromide, m.p. 198-200°C.

Step E Synthesis of 1-(4-chlorophenyl)-1-cyclopropyl-4-(3-phenoxyphenyl)-1-butene (Compound B7)

Under an argon atmosphere a slurry of 4.2 grams (0.0076 mole) of 3-phenoxyphenyl)-propyltriphenylphosphonium bromide in 75 mL of freshly distilled tetrahydrofuran was cooled to 0°C with stirring. To this mixture was added 5.4 mL (0.0079 mole ) of a 1.55 M solution of n-butyllithium in hexanes in 0.5 mL portions using a syringe during a 20 minute period. An additional 2.0 mL (0.0031 mole) of the n-butyllithium solution was then added slowly, causing a red solution to form. This solution was allowed to warm to ambient temperature at which it was stirred for 60 minutes. This solution was again cooled to 0°C, and 1.3 grams (0.0072 mole) 4-chlorophenyl cyclopropyl ketone in 5 mL of tetrahydrofuran was added portionwise using a syringe. Upon completion of addition, the reaction mixture was allowed to warm to ambient temperature. A precipitate formed. After two hours the reaction mixture was filtered. To the filtrate was added 1 mL of water with stirring to decompose any residual n-butyllithium. The filtrate was dried over anhydrous sodium sulfate and was filtered. The filtrate was evaporated under reduced pressure, leaving a mixture of a solid and an oil as the residue. To this residue was added heptane/ethyl acetate (1:2) with stirring. A solid was removed by filtration, and the filtrate was concentrated under reduced pressure. Additional solid was removed by filtration from the concentrated solution. The filtrate was placed on a column of silica gel, eluting with 500 mL of heptane/ ethyl acetate (9:1). The appropriate fractions were

combined, and the solvent was evaporated under reduced pressure, yielding 1.2 grams of 1-(4-chlorophenyl)-1-cyclopropyl-4-(3-phenoxyphenyl)-1-butene as an oil. The nmr spectrum was consistent with the proposed structure.

| | | | |
|---|---|---|---|
| Analysis for $C_{25}H_{23}ClO$ | Calc'd:<br>Found: | C 80.09;<br>C 80.15; | H 6.18;<br>H 5.98. |

Step F Synthesis of 1-(4-chlorophenyl)-1-cyclopropyl-4-(3-phenoxyphenyl)butane (Compound 88)

This compound was prepared in a manner analogous to that of Example 4, Step E, by hydrogenation of 1.0 gram (0.0027 mole) of 1-(4-chlorophenyl)-1-cyclopropyl-4-(3-phenoxyphenyl)-1-butene in the presence of 0.35 gram of Raney nickel in 75 mL of ethanol. This procedure yielded 0.8 gram of 1-(4-chlorophenyl)-1-cyclopropyl-4-(3-phenoxyphenyl)butane as an a oil. The nmr spectrum was consistent with the proposed structure.

In accordance with the composition aspect of the invention, the compounds are generally not applied full strength but are typically applied as formulations which may be applied as such or further diluted for application. Typical formulations include compositions of the active ingredient in combination with one or more agriculturally acceptable adjuvants, carriers or diluents, preferably with a surface active agent, and optionally with other active ingredients. Suitable formulations include solid compositions such as dusts, wettable powders, and granules or liquid compositions such as solutions, dispersions, suspensions, and emulsifiable concentrates, the choice varying with the type of pest and environmental factors present at the particular locus of infestation.

A typical formulation may vary widely in concentration of active ingredient and other ingredients depending upon the particular agent used, the additives and carriers used, other active ingredients, the desired mode of application, and numerous other factors well known to those skilled in formulating compositions for use in agriculture.

With due consideration to these factors, the active ingredient of a typical formulation may, for example, comprise 0.01 percent to 1 percent by weight up to about 95 percent by weight, preferably 1 percent up to 90 or 95 percent by weight, of the formulation. Agriculturally acceptable carriers, diluents, adjuvants, surface active agents, and optionally other suitable active ingredients comprise the balance of the formulation. Thus a typical formulation may contain from 0.01 to 95 (preferably 1 to 95) percent by weight active ingredient, from 0 to 30 percent by weight surface active agent, and from 5 to 99.99 (preferably 5 to 99) percent by weight of an inert agriculturally acceptable carrier or diluent.

Provided below is a general description of exemplary types of formulations which may be employed for application of the compounds of the present invention.

## SOLID OR DRY FORMULATIONS

Dry formulations are mixtures of a liquid or solid active ingredient with a solid carrier to form a particulate product comprising discrete solid particles of various sizes. Solid or dry compositions may take the form of dusts, wettable powders and granules having average particle sizes varying from about 5 microns to about 5000 microns. These compositions employ solid or dry carriers and/or diluents which may be selected from one or more of the following:

1. Attapulgite Clay: Characterized as hydrated aluminum-magnesium silicate, with or without free water, and possessing sorptive capacity of at least 35% w/w.
2. Kaolin or Kaolinite Clay: Characterized as hydrated aluminum silicate, and including the species dickite, nakrite, and halloysite, and further characterized by having low values for cation exchange capacity.
3. Montmorillonite: Characterized as hydrous aluminum silicate derived by natural modification of mica and pyrophyllite, and further sub-divided into swelling (sodium form) and non-swelling (calcium form).
4. Pyrophyllite (Talc): Characterized as hydrous magnesium or aluminum silicate and having neutral to basic pH, and further characterized by low to moderate sorptive capacity.
5. Diatomite: Class of opaline silica skeletal remains of aquatic species which includes diatomaceous earth, tripolite, kieselguhr, and fossil flour, characterized by high (85-93%) silica content, and having high absorptive and low adsorptive capacity.
6. Silica: Diverse origin materials characterized by very high (98-100%) silica content and high (75-100%) sorptive capacity (synthetic), or low sorptive capacity, such as sand.
7. Botanicals: Any material of plant origin capable of being processed into particles of the desired size, including nut shell flours, wood and cellulose flours, corncobs, and the like.
8. Calcium Carbonate

Dust formulations are finely divided solid compositions of active ingredient in admixture with a solid carrier. In most cases dust formulations have an average particle size of less than about 50 microns, typically 5 to 40 microns, an active ingredient content of 1 to 30 percent by weight, and from 70 to 99 percent by weight of one or more of the solid diluents or carriers described above. Since dust formulations are generally applied as such or mixed with other solids for application, they generally do not require a surface active agent or other adjuvants. The following exemplify typical dust formulations:

| 1% Dust | % W/W |
|---|---|
| Active Ingredient | 1.0 |
| Finely Divided Silica | 99.0 |
| | 100.0 |

| 10% Dust | % W/W |
|---|---|
| Active Ingredient | 10.0 |
| Kaolin | 90.0 |
| | 100.0 |

| 30% Dust | |
|---|---|
| Active Ingredient | 30.0 |
| Montmorillonite | 30.0 |
| Talc | 40.0 |
| | 100.0 |

Wettable powders are finely divided solid compositions which disperse readily in water or other liquid vehicles. The wettable powder may be applied as a dry dust or as a dispersion in water or other liquid. Thus, wettable powders are essentially a dust or powder formulation containing a surface active agent in addition to the active ingredient and solid carrier normally employed in dusts.

A wettable powder may thus typically contain from 1 to 95 percent by weight active ingredient, from 1 to 15 percent surface active agent, and from 4 to 98 percent by weight of one or more of the inert solid or dry carriers or diluents described above.

Suitable surface active agents may be selected from the following:
1. Salts or esters of sulfated or sulfonated fatty acids.
2. Salts or esters of ethylene oxide condensates of sulfated or sulfonated fatty acids.
3. Salts or amine derivatives of various resin and fatty acids including, but not restricted to, palmitic and myristic acids, tall oils, and taurine.
4. Salts of alkylarylsulfonates including alkylnaphthalenesulfonates and dialkylnaphthalenesulfonates.
5. Ethylene oxide condensates of mixed fatty and resin acids.
6. Ethylene oxide condensates of linear or branched chain glycols, secondary alcohols, or alkylaryl alcohols.
7. Mixed ethylene oxide and propylene oxide condensates of linear and branched chain glycols.
8. Salts of sulfonated naphthalene-formaldehyde condensates.
9. Salts of carboxylated poly-electrolytes.
10. Salts of polymerized alkylnaphthalenesulfonic acids.
11. Salts of lignin sulfonates.
12. Fatty alcohol polyglycol ethers.
13. Materials of classes 1, 2, 5, 6, 7 above when sorbed onto a sorptive, water compatible carrier.
14. Inorganic salts such as tripolyphosphate and hexametaphosphate.
15. Salts and esters of orthophosphoric acid.

16. Fatty acid esters of sorbitan.
17. Ethylene oxide condensates with fatty acid esters of sorbitan.
18. Alkylated alkene mono- and polyhydric alcohols.
19. Sulfonated castor oil.
20. Ethylene oxide condensate with lanolin.
21. Coconut alkanolamides.
22. Sulfated sperm oil.
23. Salts of linear alkyl sulfonates.
24. Tall oil ethoxylates.

The following are typical wettable powders:

| 1% Powder | % W/W |
|---|---|
| Active Ingredient | 1.0 |
| Sodium lignosulfonate | 7.5 |
| Sodium laurylsulfate | 1.5 |
| Talc | 96.0 |
| Total | 100.0 |

| 5% Powder | |
|---|---|
| Active Ingredient | 5.0 |
| Sodium lignosulfonate | 1.5 |
| Sodium alkylnaphthylene sulfonate | 1.5 |
| Attaclay | 92.0 |
| Total | 100.0 |

| 25% Powder | |
|---|---|
| Active Ingredient | 25.0 |
| Sodium lignosulfonate | 1.5 |
| Sodium laurylsulfate | 1.5 |
| Montmorillonite | 72.0 |
| Total | 100.0 |

| 90% Powder | |
|---|---|
| Active Ingredient | 90.0 |
| Sodium dibutylnaphthalenesulfonate | 0.5 |
| Sodium lignosulfonate | 3.5 |
| Kaolin clay | 6.0 |
| Total | 100.0 |

Granules are solid or dry compositions of active ingredient deposited on or in a large particle. Granules usually have an average particle size in the range of 150 to 5000 microns, typically 425 to 850 microns. Granular formulations generally contain from 1 to 50 percent by weight of active ingredient, from 1 to 15 percent by weight of one or more of the surface active agents described above, and from 50 to 98 percent by weight of one or more of the inert solid or dry carriers of diluents described above.

Granular formulations may be of several types. Impregnated granules are those in which the active ingredient is applied, normally as a solution, to large particles of an absorbent diluent or carrier such as attapulgite or kaolin clay, corncobs or expanded mica. Surface coated granules are granules produced by adhering an active ingredient in finely divided form on the surface of a generally non-absorbent particle or by applying a solution of active ingredient to the surface of such a carrier. The carrier or core may be water

soluble, such as prilled fertilizer or urea, or insoluble, such as sand, marble chips, corncobs, or coarse talc, as described above. Particularly useful are granules wherein a wettable powder is adhered as a surface coating to a sand or other insoluble particle, so that the wettable powder may be dispersed on contact of the granule with moisture. Granules may also be produced by agglomeration of dusts or powders, by compaction, by extrusion through a die, or by use of a granulation disk.

The following are typical granular formulations:

| 1% Granule | % W/W |
|---|---|
| Active Ingredient | 1.0 |
| Attapulgite | 99.0 |
| Total | 100.0 |

| 5% Granule | |
|---|---|
| Active Ingredient | 5.0 |
| Attapulgite | 95.0 |
| Total | 100.0 |

The granules above may be prepared by dissolving the active ingredient in a volatile solvent such as methylene chloride, coating large particles of attapulgite clay with the solution, then allowing the solvent to evaporate.

As indicated above, granules may also be adhered to a nonabsorbent core material. The following are typical formulations:

| 5% Sand-Core Granule | % W/W |
|---|---|
| 75% Powder Base | 6.64 |
| Active compound | 75.0 |
| Sodium alkylnaphthalenesulfonate | 1.0 |
| Sodium lignosulfonate | 4.0 |
| Barden clay | 20.0 |
| Dilute Polyvinylacetate | 1.75 |
| Silica (425-850) | 91.61 |
| Total | 100.00 |

| 47.5% Sand-Core Granule | % W/W |
|---|---|
| 95% Powder Base | 50.0 |
| Active compound | 95.0 |
| Sodium alkylnaphthalenesulfonate | 1.0 |
| Sodium lignosulfonate | 4.0 |
| Dilute Polyvinylacetate adherent | 2.0 |
| Silica (425-850) | 48.00 |
| Total | 100.00 |

The foregoing sand-core granules may be prepared by incorporating the active compound into the base, then adhering the base to sand, utilizing an adhesive such as polyvinylacetate to assure adhesion.

LIQUID AND SEMI-LIQUID FORMULATIONS

Liquid formulations are those which contain the active ingredient dissolved or dispersed in one or more inert liquid carriers or diluents, containing from 0.01 to about 95% active ingredient. Carriers suitable for use in liquid formulations may be selected from the following:

1. Water.
2. Aliphatic petroleum solvents including kerosene, light refined mineral oils, and diesel oils.
3. Aromatic petroleum solvents including coal tar fractions yielding xylene, toluene, and benzene; light, medium, and heavy aromatic naphthas; and alkylated mixed naphthenics.
4. Alcohols such as ethanol and isopropyl alcohol.
5. Alkyl ethers of glycols.
6. Esters including dibutyl phthalate, di-2-ethylhexyl phthalate, and ethyl acetate.
7. Ketones including cyclohexanone, methyl isobutyl ketone, acetone, diacetone, and isophorone.
8. Chlorinated hydrocarbons including ethylene dichloride, methylene chloride, chlorobenzene, chlorinated toluene, and chlorinated xylene.
9. Vegetable oils including cottonseed, soybean, pine, sesame, and palm oils.
10. Aqueous solutions of natural origin such as liquors obtained in processing natural sugar products, and fermentation broths.

Solutions are liquid compositions containing from about 0.01 to 95 percent by weight active ingredient and from 1 to 99.99 percent by weight of one or more of the inert liquid diluents or carriers described above. These may be applied as such or further diluted for application.

Suspensions or dispersions (also sometimes called flowable formulations) are liquid formulations containing from 0.01 to 95 percent by weight active ingredient and from 1 to 99.99 percent by weight of an inert liquid diluent or carrier, in which the active ingredient is wholly or partially insoluble in the diluent or carrier at the concentration level employed. Suspension or dispersion is frequently facilitated by incorporating from 1 to 30 percent by weight of one or more surface active agents described above, alone or together with a thickener or suspending agent. Like solutions, dispersions may be used as such or further diluted with a liquid carrier for application.

The following illustrate suspensions suitable for use in the present invention:

| 25% Oil Suspension: | % W/W |
|---|---|
| Active ingredient | 25.0 |
| polyoxyethylene sorbitol hexaoleate | 5.0 |
| aliphatic hydrocarbon oil | 70.0 |
| Total | 100.0 |

| 1% Aqueous Suspension: | % W/W |
|---|---|
| Active ingredient | 1.0 |
| Polyacrylic acid thickener | 0.3 |
| Sodium alkylnaphthalenesulfonate | 1.0 |
| Sodium lignosulfonate | 4.0 |
| Polyvinyl alcohol suspending agent | 1.0 |
| Water | 92.7 |
| Total | 100.0 |

| 20% Aqueous Suspension: | |
|---|---|
| Active ingredient | 20.0 |
| Polyacrylic acid thickener | 0.3 |
| Sodium alkylnaphthalenesulfonate | 1.0 |
| Sodium lignosulfonate | 4.0 |
| Polyvinyl alcohol suspending agent | 1.0 |
| Water | 73.7 |
| Total | 100.0 |

| 40% Aqueous Suspension: | |
|---|---|
| Active ingredient | 40.0 |
| Polyacrylic acid thickener | 0.3 |
| Dodecylphenol polyethylene glycol ether | 0.5 |
| Disodium phosphate | 1.0 |
| Monosodium phosphate | 0.5 |
| Polyvinyl alcohol | 1.0 |
| Water | 56.7 |
| Total | 100.0 |

Emulsifiable concentrates (EC's) are homogeneous liquid compositions, containing the active ingredient dissolved in a liquid carrier. Commonly used liquid carriers include xylene, heavy aromatic naphthas, isophorone, and other nonvolatile or slightly volatile organic solvents. For application these concentrates are dispersed in water, or other liquid vehicle, forming an emulsion, and are normally applied as a spray to the area to be treated. The concentration of the essential active ingredient in EC's may vary according to the manner in which the composition is to be applied, but, in general, is in the range of 0.01 to 95 percent by weight of active ingredient. Also included in the composition are from 1 to 30 percent by weight surface active agent and from 4 to 97.99 percent of one or more of the inert liquid carriers described above. The following are typical EC compositions:

| 1% Emulsifiable Concentrate | % W/W |
|---|---|
| Active Ingredient | 1.0 |
| Anionic calcium dodecylbenzenesulfonate | 4.2 |
| Nonionic polyethoxylated nonylphenol (Mol. Wt. 450-500) | 0.4 |
| Nonionic polyethoxylated nonylphenol (Mol. Wt. 1400-1600) | 1.1 |
| Nonionic paste of 100% polyalkylene glycol ether | 0.4 |
| Xylene | 92.9 |
| Total | 100.0 |

| 5% Emulsifiable Concentrate | |
|---|---|
| Active Ingredient | 5.0 |
| Anionic calcium dodecylbenzene sulfonate | 4.2 |
| Nonionic polyethoxylated nonylphenol (Mol. Wt. 450-500) | 0.4 |
| Nonionic polyethoxylated nonylphenol (Mol. Wt. 1450-1600) | 1.1 |
| Nonionic paste of 100% poly alkylene glycol ether | 0.4 |
| Xylene | 88.9 |
| Total | 100.0 |

| 10% Emulsifiable Concentrate | % W/W |
|---|---|
| Active Ingredient | 10.0 |
| Blend of alkylnaphthalenesulfonate and polyoxyethylene ethers | 4.0 |
| Xylene | 86.0 |
| Total | 100.0 |

| 50% Emulsifiable Concentrate | |
|---|---|
| Active Ingredient | 50.0 |
| Blend of alkylnaphthalenesulfonate and polyoxyethylene ethers | 6.0 |
| Epoxidized soybean oil | 1.0 |
| Xylene | 43.0 |
| Total | 100.0 |

| 75% Emulsifiable Concentrate | |
|---|---|
| Active Ingredient | 75.0 |
| Blend of alkylnaphthalenesulfonate and polyoxyethylene ethers | 4.0 |
| Xylene | 21.0 |
| Total | 100.0 |

Other useful formulations include simple solutions of the active ingredient in a relatively non-volatile solvent such as corn oil, kerosene, propylene glycol, or other organic solvents. This type of formulation is particularly useful for ultra low volume application.

The concentration of the active ingredient in use dilution is normally in the range of about 2% to about 0.1%. Many variations of spraying, dusting, and controlled or slow release compositions in the art may be used by substituting or adding a compound of this invention into compositions known or apparent to the art.

These compositions may be formulated and applied with other suitable active ingredients, including nematicides, insecticides, acaricides, fungicides, plant regulators, herbicides, fertilizers, etc.

In applying the foregoing chemicals, an effective insect controlling amount of active ingredient must be applied, sometimes referred to herein as an insecticidal amount. While the application rate will vary widely depending on the choice of compound, the formulation and mode of application, the plant species being protected and the planting density, a suitable use rate may be in the range of 0.10 to 0.50 kg per hectare, preferably 0.25 to about 1.5 kg/hectare.

The compounds of this invention may be applied by incorporating or applying a formulation thereof to a food source for the insects to be controlled, i.e. the locus where control is required, including application to the above ground portions of plants on which the insects feed, to the soil in which plants are or are about to be planted in order to provide control of soil-borne insects, or in a bait-type formulation for application to surfaces on which insects normally do not feed. When applying the compounds to the soil, the compounds may be broadcast broadly over the planted area or the area to be planted or by limiting the application to a small area or band in the root zone where plants are or are to be planted. When either method of soil application is used, sufficient compound must be applied to provide an insect controlling concentration of the compound in the soil in the root zone. For the present a suitable concentration is about 0.2 to about 50 parts by weight of compound per million parts of soil.

The insecticidal activity of the pyrethroid-like compound of this invention was evaluated as follows:

Foliar Evaluation

The compound was tested by foliar application at various concentrations in aqueous solutions containing 10% acetone and 0.25% octyl phenoxypolyethoxy ethanol. The evaluation utilized Mexican bean beetle (Epilachna varivestis), southern armyworm (Spodoptera eridania), pea aphid (Acyrthosiphon pisum), cabbage looper (Trichonlusia ni), beet armyworm (Spodoptera exigua), and twospotted spider mite (Tetranychus urticae).

For all insects except pea aphid, pinto bean (Phaseolus vulgaris) plants were placed on a revolving turntable in a hood, and the test solutions were applied with a sprayer. The test solutions were applied to the upper and lower surfaces of the plant leaves to runoff. The plants were then allowed to dry and were severed at the base of the stem before being placed in cups. Ten individuals of the appropriate insect species were placed in each cup and the cup covered. Mortality was read 48 hours later.

Fava bean was substituted for pinto bean in the case of pea aphid, and the treated, potted plants were placed in cups infested with ten individuals and covered. Mortality was read 48 hours later.

Acaricidal tests were performed using the following procedure: Leaves infested with adult twospotted spider mites (Tetranychus urticae) were removed from culture plants and cut into segments containing 50-75 female mites. Each segment was placed on the upper leaf surface of a whole pinto bean plant. After the mites had migrated to the under surfaces of the leaves, the leaf segments used to infest were removed, and each plant was sprayed with test chemical as described above. After the plants had dried, the entire plant and pot were placed in metal trays in a hood, a supply of water in the tray keeping the plants turgid. After 48 hours the living and dead mites were counted, and percent mortality was calculated.

The results of these tests are shown in Table 4.

Soil Evaluation

A stock solution of the test compound was prepared by dissolving 9.6 mg in 10 mL of acetone and diluting with 90 mL of acetone/water (1:9). The addition of 5 mL of this stock solution to 30 grams of air-dried, clay loam soil in a three ounce plastic cup provided a concentration of 16 ppm of the test compound in the soil. Serial dilution of the stock solution was used to provide concentrations of the test compound in soil of 8, 4, 2, 1, 0.5, and 0.25 ppm. In all cases 5 mL of a solution having the required concentration was added to 30 grams of soil. The treated soil was allowed to stand uncovered in a hood for 0.5 hour to evaporate the acetone. Before infesting the soil with southern corn rootworm larvae (Diabrotica undecimpunctata howardi Barber) the soil was mixed thoroughly, and two three-day-old corn sprouts were planted in it. Ten early third-stage (9-10 days old) southern corn rootworm larvae were placed in the cup which was covered with a plastic bag. After storage at 74-78°F for 48 hours, the mortality of the larvae was determined by removing the cup from the plastic bag, removing the cover, and placing the cup in a modified Berlese polyethylene funnel fitted with an 18-mesh screen. The funnels were placed over containers of an aqueous detergent solution. Incandescent lights (100 watts) were placed 36 cm above the soil samples. The heat from these lights slowly dried the soil causing larvae that had not been affected by the test compound to emerge from the soil and drop into the detergent solution. The percent mortality was determined in this manner for each concentration.

Results of these tests are reported in Table 5.

Fish Toxicity

The toxicity towards fish was determined in a 48 hour static bioassay using the bluegill sunfish (Lepomis macrochirus). Three fish ranging in size from 1 to 2 inches were placed in a 0.95 liter jar containing the specified concentration of the compound. Two replicates were used for each concentration. After 48 hours the percent kill was determined. Concentrations of chemicals used were 6.3 ppm, 3.1 ppm, and occasionally 1.7 ppm. Compound 16, the compound of Example 1 and a preferred compound of this invention, exhibited 83% kill at 6.3 ppm. Another preferred compound, Compound 24, killed only 50% of the fish at the same concentration. By way of comparison, cypermethrin, a conventional pyrethroid insecticide used widely for crop protection, displays 100% kill at a concentration of 0.01 ppm.

The remarkably low toxicity towards fish of the pyrethroid-like compounds is certainly unexpected, and this factor, in combination with the demonstrated insecticidal activity, should make them appropriate compounds for control of insect infestations in aquatic environments, such as rice paddies.

TABLE 1 - TABLE OF ETHERS, THIOETHERS, AND BUTANE DERIVATIVES

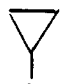

$Ar-CH-CH_2-Z-CH_2-Ar'$

| Cmpd No. | Ar | Z | Ar' |
|---|---|---|---|
| 1 | phenyl | O | 2-methyl[1,1'-biphenyl]-3-yl |
| 2 | phenyl | O | 3-phenoxyphenyl |
| 3 | phenyl | O | 4-fluoro-3-phenoxyphenyl |
| 4 | 4-fluorophenyl | O | 2-methyl[1,1'-biphenyl]-3-yl |
| 5 | 4-fluorophenyl | O | 3-phenoxyphenyl |
| 6 | 4-fluorophenyl | O | 4-fluoro-3-phenoxyphenyl |
| 7 | 2-chlorophenyl | O | 2-methyl[1,1'-biphenyl]-3-yl |
| 8 | 2-chlorophenyl | O | 3-phenoxyphenyl |
| 9 | 2-chlorophenyl | O | 4-fluoro-3-phenoxyphenyl |
| 10 | 3-chlorophenyl | O | 2-methyl[1,1'-biphenyl]-3-yl |
| 11 | 3-chlorophenyl | O | 3-phenoxyphenyl |
| 12 | 3-chlorophenyl | O | 4-fluoro-3-phenoxyphenyl |
| 13 | 4-chlorophenyl | O | 2-methyl[1,1'-biphenyl]-3-yl |
| 14 | 4-chlorophenyl | O | 3-phenoxyphenyl |
| 15 | 4-chlorophenyl | O | 3-phenoxyphenyl (Stereoisomer B)[a] |
| 16 | 4-chlorophenyl | O | 4-fluoro-3-phenoxyphenyl |
| 17 | 4-chlorophenyl | O | 4-fluoro-3-phenoxyphenyl (Stereoisomer A)[b] |
| 18 | 4-chlorophenyl | O | 4-fluoro-3-phenoxyphenyl (Stereoisomer B)[c] |
| 19 | 4-chlorophenyl | S | 2-methyl[1,1'-biphenyl]-3-yl |
| 20 | 4-chlorophenyl | S | 3-phenoxyphenyl |
| 21 | 4-chlorophenyl | S | 4-fluoro-3-phenoxyphenyl |
| 22 | 4-bromophenyl | O | 2-methyl[1,1'-biphenyl]-3-yl |
| 23 | 4-bromophenyl | O | 3-phenoxyphenyl |

Table 1 (continued)

| Cmpd No. | Ar | Z | Ar' |
|---|---|---|---|
| 24 | 4-bromophenyl | O | 4-fluoro-3-phenoxyphenyl |
| 25 | 4-methylphenyl | O | 2-methyl[1,1'-biphenyl]-3-yl |
| 26 | 4-methylphenyl | O | 3-phenoxyphenyl |
| 27 | 4-methylphenyl | O | 4-fluoro-3-phenoxyphenyl |
| 28 | 3-ethylphenyl | O | 2-methyl[1,1'-biphenyl]-3-yl |
| 29 | 3-ethylphenyl | O | 3-phenoxyphenyl |
| 30 | 3-ethylphenyl | O | 4-fluoro-3-phenoxyphenyl |
| 31 | 4-ethylphenyl | O | 2-methyl[1,1'-biphenyl]-3-yl |
| 32 | 4-ethylphenyl | O | 3-phenoxyphenyl |
| 33 | 4-ethylphenyl | O | 4-fluoro-3-phenoxyphenyl |
| 34 | 4-_t_-butylphenyl | O | 2-methyl[1,1'-biphenyl]-3-yl |
| 35 | 4-_t_-butylphenyl | O | 3-phenoxyphenyl |
| 36 | 4-_t_-butylphenyl | O | 4-fluoro-3-phenoxyphenyl |
| 37 | 4-trifluoromethylphenyl | O | 2-methyl[1,1'-biphenyl]-3-yl |
| 38 | 4-trifluoromethylphenyl | O | 3-phenoxyphenyl |
| 39 | 4-trifluoromethylphenyl | O | 4-fluoro-3-phenoxyphenyl |
| 40 | 4-methoxyphenyl | O | 2-methyl[1,1'-biphenyl]-3-yl |
| 41 | 4-methoxyphenyl | O | 3-phenoxyphenyl |
| 42 | 4-methoxyphenyl | O | 4-fluoro-3-phenoxyphenyl |
| 43 | 4-ethoxyphenyl | O | 2-methyl[1,1'-biphenyl]-3-yl |
| 44 | 4-ethoxyphenyl | O | 3-phenoxyphenyl |
| 45 | 4-ethoxyphenyl | O | 4-fluoro-3-phenoxyphenyl |
| 46 | 4-difluoromethoxyphenyl | O | 2-methyl[1,1'-biphenyl]-3-yl |
| 47 | 4-difluoromethoxyphenyl | O | 3-phenoxyphenyl |
| 48 | 4-difluoromethoxyphenyl | O | 4-fluoro-3-phenoxyphenyl |
| 49 | 4-trifluoromethoxyphenyl | O | 2-methyl[1,1'-biphenyl]-3-yl |
| 50 | 4-trifluoromethoxyphenyl | O | 3-phenoxyphenyl |
| 51 | 4-trifluoromethoxyphenyl | O | 4-fluoro-3-phenoxyphenyl |
| 52 | 4-(2-fluoroethoxy)phenyl | O | 2-methyl[1,1'-biphenyl]-3-yl |
| 53 | 4-(2-fluoroethoxy)phenyl | O | 3-phenoxyphenyl |
| 54 | 4-(2-fluoroethoxy)phenyl | O | 4-fluoro-3-phenoxyphenyl |
| 55 | 4-trifluoromethylthiophenyl | O | 2-methyl[1,1'-biphenyl]-3-yl |
| 56 | 4-trifluoromethylthiophenyl | O | 3-phenoxyphenyl |

Table 1 (continued)

| Cmpd No. | Ar | Z | Ar' |
|---|---|---|---|
| 57 | 4-trifluoromethylthiophenyl | O | 4-fluoro-3-phenoxyphenyl |
| 58 | 4-trifluoromethylsulfinylphenyl | O | 2-methyl[1,1'-biphenyl]-3-yl |
| 59 | 4-trifluoromethylsulfinylphenyl | O | 3-phenoxyphenyl |
| 60 | 4-trifluoromethylsulfinylphenyl | O | 4-fluoro-3-phenoxyphenyl |
| 61 | 4-trifluoromethylsulfonylphenyl | O | 2-methyl[1,1'-biphenyl]-3-yl |
| 62 | 4-trifluoromethylsulfonylphenyl | O | 3-phenoxyphenyl |
| 63 | 4-trifluoromethylsulfonylphenyl | O | 4-fluoro-3-phenoxyphenyl |
| 64 | 1,3-benzodioxol-5-yl | O | 2-methyl[1,1'-biphenyl]-3-yl |
| 65 | 1,3-benzodioxol-5-yl | O | 3-phenoxyphenyl |
| 66 | 1,3-benzodioxol-5-yl | O | 4-fluoro-3-phenoxyphenyl |
| 67 | 2,2-difluoro-1,3-benzodioxol-5-yl | O | 2-methyl[1,1'-biphenyl]-3-yl |
| 68 | 2,2-difluoro-1,3-benzodioxol-5-yl | O | 3-phenoxyphenyl |
| 69 | 2,2-difluoro-1,3-benzodioxol-5-yl | O | 4-fluoro-3-phenoxyphenyl |
| 70 | 3-chloro-4-methoxyphenyl | O | 2-methyl[1,1'-biphenyl]-3-yl |
| 71 | 2,3-dihydro-2,2-dimethylbenzofuran-5-yl | O | 2-methyl[1,1'-biphenyl]-3-yl |
| 72 | 2,3-dihydro-2,2-dimethylbenzofuran-5-yl | O | 3-phenoxyphenyl |
| 73 | 2,3-dihydro-2,2-dimethylbenzofuran-5-yl | O | 4-fluoro-3-phenoxyphenyl |
| 74 | 2,2,3,3-tetrafluorobenzofuran-5-yl | O | 2-methyl[1,1'-biphenyl]-3-yl |
| 75 | 2,2,3,3-tetrafluorobenzofuran-5-yl | O | 3-phenoxyphenyl |
| 76 | 2,2,3,3-tetrafluorobenzofuran-5-yl | O | 4-fluoro-3-phenoxyphenyl |
| 77 | 2-thienyl | O | 2-methyl[1,1'-biphenyl]-3-yl |
| 78 | 2-thienyl | O | 3-phenoxyphenyl |
| 79 | 2-thienyl | O | 4-fluoro-3-phenoxyphenyl |
| 80 | 4-chlorophenyl | O | 6-phenoxy-2-pyridyl |

Table 1 (continued)

| Cmpd No. | Ar | Z | Ar' |
|---|---|---|---|
| 81 | 4-ethoxyphenyl | O | 6-phenoxy-2-pyridyl |
| 82 | 2-chlorophenyl | $CH_2$ | 3-phenoxyphenyl |
| 83 | 2-chlorophenyl | $CH_2$ | 4-fluoro-3-phenoxyphenyl |
| 84 | 3-chlorophenyl | $CH_2$ | 2-methyl[1,1'-biphenyl]-3-yl |
| 85 | 3-chlorophenyl | $CH_2$ | 3-phenoxyphenyl |
| 86 | 3-chlorophenyl | $CH_2$ | 4-fluoro-3-phenoxyphenyl |
| 87 | 4-chlorophenyl | $CH_2$ | 2-methyl[1,1'-biphenyl]-3-yl |
| 88 | 4-chlorophenyl | $CH_2$ | 3-phenoxyphenyl |
| 89 | 4-chlorophenyl | $CH_2$ | 4-fluoro-3-phenoxyphenyl |
| 90 | 4-chlorophenyl | $CH_2$ | 6-phenoxy-2-pyridyl |
| 91 | 4-bromophenyl | $CH_2$ | 2-methyl[1,1'-biphenyl]-3-yl |
| 92 | 4-bromophenyl | $CH_2$ | 3-phenoxyphenyl |
| 93 | 4-bromophenyl | $CH_2$ | 4-fluoro-3-phenoxyphenyl |
| 94 | 4-methylphenyl | $CH_2$ | 2-methyl[1,1'-biphenyl]-3-yl |
| 95 | 4-methylphenyl | $CH_2$ | 3-phenoxyphenyl |
| 96 | 4-methylphenyl | $CH_2$ | 4-fluoro-3-phenoxyphenyl |
| 97 | 4-trifluoromethylphenyl | $CH_2$ | 2-methyl[1,1'-biphenyl]-3-yl |
| 98 | 4-trifluoromethylphenyl | $CH_2$ | 3-phenoxyphenyl |
| 99 | 4-trifluoromethylphenyl | $CH_2$ | 4-fluoro-3-phenoxyphenyl |
| 100 | 4-methoxyphenyl | $CH_2$ | 2-methyl[1,1'-biphenyl]-3-yl |
| 101 | 4-methoxyphenyl | $CH_2$ | 3-phenoxyphenyl |
| 102 | 4-methoxyphenyl | $CH_2$ | 4-fluoro-3-phenoxyphenyl |
| 103 | 4-ethoxyphenyl | $CH_2$ | 2-methyl[1,1'-biphenyl]-3-yl |
| 104 | 4-ethoxyphenyl | $CH_2$ | 3-phenoxyphenyl |
| 105 | 4-ethoxyphenyl | $CH_2$ | 4-fluoro-3-phenoxyphenyl |
| 106 | 4-difluoromethoxyphenyl | $CH_2$ | 2-methyl[1,1'-biphenyl]-3-yl |
| 107 | 4-difluoromethoxyphenyl | $CH_2$ | 3-phenoxyphenyl |
| 108 | 4-difluoromethoxyphenyl | $CH_2$ | 4-fluoro-3-phenoxyphenyl |
| 109 | 4-trifluoromethoxyphenyl | $CH_2$ | 2-methyl[1,1'-biphenyl]-3-yl |
| 110 | 4-trifluoromethoxyphenyl | $CH_2$ | 3-phenoxyphenyl |
| 111 | 4-trifluoromethoxyphenyl | $CH_2$ | 4-fluoro-3-phenoxyphenyl |
| 112 | 4-(2-fluoroethoxy)phenyl | $CH_2$ | 2-methyl[1,1'-biphenyl]-3-yl |
| 113 | 4-(2-fluoroethoxy)phenyl | $CH_2$ | 3-phenoxyphenyl |

Table 1 (continued)

| Cmpd No. | Ar | Z | Ar' |
|---|---|---|---|
| 114 | 4-(2-fluoroethoxy)phenyl | $CH_2$ | 4-fluoro-3-phenoxyphenyl |
| 115 | 1,3-benzodioxol-5-yl | $CH_2$ | 2-methyl[1,1'-biphenyl]-3-yl |
| 116 | 1,3-benzodioxol-5-yl | $CH_2$ | 3-phenoxyphenyl |
| 117 | 1,3-benzodioxol-5-yl | $CH_2$ | 4-fluoro-3-phenoxyphenyl |
| 118 | 2,2-difluoro-1,3-benzo-dioxol-5-yl | $CH_2$ | 2-methyl[1,1'-biphenyl]-3-yl |
| 119 | 2,2-difluoro-1,3-benzo-dioxol-5-yl | $CH_2$ | 3-phenoxyphenyl |
| 120 | 2,2-difluoro-1,3-benzo-dioxol-5-yl | $CH_2$ | 4-fluoro-3-phenoxyphenyl |
| 121 | 4-trifluoromethylthiophenyl | $CH_2$ | 2-methyl[1,1'-biphenyl]-3-yl |
| 122 | 4-trifluoromethylthiophenyl | $CH_2$ | 3-phenoxyphenyl |
| 123 | 4-trifluoromethylthiophenyl | $CH_2$ | 4-fluoro-3-phenoxyphenyl |
| 124 | 2,3-dihydro-2,2-dimethyl-benzofuran-5-yl | $CH_2$ | 2-methyl[1,1'-biphenyl]-3-yl |
| 125 | 2,3-dihydro-2,2-dimethyl-benzofuran-5-yl | $CH_2$ | 3-phenoxyphenyl |
| 126 | 2,3-dihydro-2,2-dimethyl benzofuran-5-yl | $CH_2$ | 4-fluoro-3-phenoxyphenyl |
| 127 | 2,2,3,3-tetrafluoro-benzofuran-5-yl | $CH_2$ | 2-methyl[1,1'-biphenyl]-3-yl |
| 128 | 2,2,3,3-tetrafluoro-benzofuran-5-yl | $CH_2$ | 3-phenoxyphenyl |
| 129 | 2,2,3,3-tetrafluoro-benzofuran-5-yl | $CH_2$ | 4-fluoro-3-phenoxyphenyl |
| 130 | 2-thienyl | $CH_2$ | 3-phenoxyphenyl |
| 131 | 2-thienyl | $CH_2$ | 4-fluoro-3-phenoxyphenyl |
| 132 | 4-ethoxyphenyl | $CH_2$ | 6-phenoxy-2-pyridyl |

a. $[\alpha]_D^{25}$=(-)26.20° in $CHCl_3$

b. $[\alpha]_D^{25}$=(+)22.19° in $CHCl_3$

c. $[\alpha]_D^{25}$=(-)20.64° in $CHCl_3$

TABLE 2 - INSECTICIDAL AND ACARICIDAL 1,4-DIARYL-1-CYCLOPROPYL-1,3-BUTADIENE DERIVATIVES

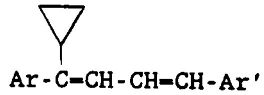

| Cmpd No. | Ar | Ar' |
|---|---|---|
| A1 | 3-chlorophenyl | 2-methyl[1,1'-biphenyl]-3-yl |
| A2 | 3-chlorophenyl | 3-phenoxyphenyl |
| A3 | 3-chlorophenyl | 4-fluoro-3-phenoxyphenyl |
| A4 | 4-chlorophenyl | 2-methyl[1,1'-biphenyl]-3-yl |
| A5 | 4-chlorophenyl | 3-phenoxyphenyl |
| A6 | 4-chlorophenyl | 4-fluoro-3-phenoxyphenyl |
| A7 | 4-chlorophenyl | 6-phenoxy-2-pyridyl |
| A8 | 4-methylphenyl | 2-methyl[1,1'-biphenyl]-3-yl |
| A9 | 4-methylphenyl | 3-phenoxyphenyl |
| A10 | 4-methylphenyl | 4-fluoro-3-phenoxyphenyl |
| A11 | 4-trifluoromethylphenyl | 2-methyl[1,1'-biphenyl]-3-yl |
| A12 | 4-trifluoromethylphenyl | 3-phenoxyphenyl |
| A13 | 4-trifluoromethylphenyl | 4-fluoro-3-phenoxyphenyl |
| A14 | 4-ethoxyphenyl | 2-methyl[1,1'-biphenyl]-3-yl |
| A15 | 4-ethoxyphenyl | 3-phenoxyphenyl |
| A16 | 4-ethoxyphenyl | 4-fluoro-3-phenoxyphenyl |
| A17 | 4-trifluoromethoxyphenyl | 2-methyl[1,1'-biphenyl]-3-yl |
| A18 | 4-trifluoromethoxyphenyl | 3-phenoxyphenyl |
| A19 | 4-trifluoromethoxyphenyl | 4-fluoro-3-phenoxyphenyl |
| A20 | 1,3-benzodioxol-5-yl | 2-methyl[1,1'-biphenyl]-3-yl |
| A21 | 1,3-benzodioxol-5-yl | 3-phenoxyphenyl |
| A22 | 1,3-benzodioxol-5-yl | 4-fluoro-3-phenoxyphenyl |
| A23 | 2,3-dihydro-2,2-dimethyl-benzofuran-5-yl | 2-methyl[1,1'-biphenyl]-3-yl |
| A24 | 2,3-dihydro-2,2-dimethyl-benzofuran-5-yl | 3-phenoxyphenyl |

Table 2 - (continued)

| Cmpd No. | Ar | Ar' |
|---|---|---|
| A25 | 2,3-dihydro-2,2-dimethyl-benzofuran-5-yl | 4-fluoro-3-phenoxyphenyl |
| A26 | 2-thienyl | 2-methyl[1,1-biphenyl]-3-yl |
| A27 | 2-thienyl | 3-phenoxyphenyl |
| A28 | 2-thienyl | 4-fluoro-3-phenoxyphenyl |

TABLE 3 - INSECTICIDAL AND ACARICIDAL 1,4-DIARYL-CYCLOPROPYL-1-BUTENE DERIVATIVES

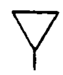

$Ar-C=CH-CH_2-CH_2-Ar'$

| Cmpd No. | Ar | Ar' |
|---|---|---|
| B1 | phenyl | 4-fluoro-3-phenoxyphenyl |
| B2 | 4-fluorophenyl | 3-phenoxyphenyl |
| B3 | 4-fluorophenyl | 4-fluoro-3-phenoxyphenyl |
| B4 | 2-chlorophenyl | 2-methyl[1,1'-biphenyl]-3-yl |
| B5 | 2-chlorophenyl | 3-phenoxyphenyl |
| B6 | 2-chlorophenyl | 4-fluoro-3-phenoxyphenyl |
| B7 | 4-chlorophenyl | 3-phenoxyphenyl |
| B8 | 4-chlorophenyl | 4-fluoro-3-phenoxyphenyl |
| B9 | 4-bromophenyl | 3-phenoxyphenyl |
| B10 | 4-ethylphenyl | 2-methyl[1,1'-biphenyl]-3-yl |
| B11 | 4-ethylphenyl | 4-fluoro-3-phenoxyphenyl |
| *B12 | 4-methoxyphenyl | 3-phenoxyphenyl |
| **B13 | 4-methoxyphenyl | 3-phenoxyphenyl |
| B14 | 4-difluoromethoxyphenyl | 2-methyl[1,1'-biphenyl]-3-yl |
| B15 | 4-difluoromethoxyphenyl | 3-phenoxyphenyl |
| B16 | 4-difluoromethoxyphenyl | 4-fluoro-3-phenoxyphenyl |
| B17 | 4-(2-fluoroethoxy)phenyl | 3-phenoxyphenyl |
| B18 | 4-(2-fluoroethoxy)phenyl | 4-fluoro-3-phenoxyphenyl |
| B19 | 4-trifluoromethylthiophenyl | 2-methyl[1,1'-biphenyl]-3-yl |
| B20 | 4-trifluoromethylthiophenyl | 3-phenoxyphenyl |
| B21 | 4-trifluoromethylthiophenyl | 4-fluoro-3-phenoxyphenyl |
| B22 | 2,2-difluoro-1,3-benzo-dioxol-5-yl | 2-methyl[1,1'-biphenyl]-3-yl |
| B23 | 2,2-difluoro-1,3-benzo-dioxol-5-yl | 3-phenoxyphenyl |
| B24 | 2,2-difluoro-1,3-benzo-dioxol-5-yl | 4-fluoro-3-phenoxyphenyl |

Table 3 (continued)

| Cmpd No. | Ar | Ar' |
|---|---|---|
| B25 | 2,2,3,3-tetrafluorobenzo-furan-5-yl | 2-methyl[1,1'-biphenyl]-3-yl |
| B26 | 2,2,3,3-tetrafluorobenzo-furan-5-yl | 3-phenoxyphenyl |
| B27 | 2,2,3,3-tetrafluorobenzo-furan-5-yl | 4-fluoro-3-phenoxyphenyl |

* Mixture of 57% Z isomer and 43% E isomer by gas chromatographic analysis (area %)

** Mixture of 86% Z isomer and 14% E isomer by gas chromatographic analysis (area %)

## TABLE 4 - FOLIAR INSECTICIDAL TEST RESULTS

| Cmpd No. | Rate (ppm) | % Kill BAW | MBB | SAW | TSM | CL | PA |
|---|---|---|---|---|---|---|---|
| 1 | 500 | | | | 9 | | 0 |
| | 100 | 45 | 0 | | | 20 | |
| 2 | 500 | | | 23 | | | 5 |
| | 100 | 95 | 95 | | | 55 | |
| 3 | 500 | | | | 20 | | 25 |
| | 100 | 100 | 100 | | | 85 | |
| 4 | 1000 | | | | | | 15 |
| | 250 | | 85 | | | 85 | |
| 5 | 500 | | | | | | 70 |
| | 250 | | 100 | | | 95 | |
| 6 | 1000 | | | | | | 60 |
| | 250 | | 100 | | | 100 | |
| 10 | 1000 | | 35 | | 29 | 95 | 50 |
| 11 | 1000 | | 100 | | 55 | 100 | 100 |
| 12 | 1000 | | 100 | | 100 | 100 | 90 |
| 13 | 1000 | | 100 | 100 | 100 | | 100 |
| | 100 | 90 | | | | 50 | |
| 14 | 1000 | | 100 | 100 | 90[a] | | 100 |
| | 100 | 100 | | | | 95 | |
| 15 | 1000 | | 100 | | | 100 | 90 |

Table 4 (continued)

| Cmpd | Rate | % Kill | | | | | |
|---|---|---|---|---|---|---|---|
| No. | (ppm) | BAW | MBB | SAW | TSM | CL | PA |
| 16 | 1000 | | 100 | 100 | 100 | | 100 |
| | 100 | 100 | | | | 100 | |
| 17 | 500 | | | | 38 | | 100 |
| | 100 | | 75 | 95 | | | |
| | 50 | 90 | | | | 95 | |
| 18 | 500 | | | | 83 | | 100 |
| | 100 | | 100 | 100 | | | |
| | 50 | 100 | | | | 100 | |
| 19 | 1000 | | | | 11 | | |
| | 500 | | 45 | | | | |
| | 250 | | | | | 100 | 20 |
| 20 | 500 | | | | 12 | | |
| | 250 | | 95 | | | 100 | 75 |
| 21 | 500 | | | | 15 | | |
| | 250 | | 100 | | | 100 | 90 |
| 22 | 1000 | 100 | 80 | | 60 | | 70 |
| | 500 | | | | | 95 | |
| 23 | 1000 | 100 | 100 | | 77 | | 100 |
| | 500 | | | | | 100 | |
| 24 | 1000 | 100 | 100 | | 100 | | 80 |
| | 500 | | | | | 100 | |

Table 4 (continued)

| Cmpd No. | Rate (ppm) | BAW | MBB | SAW | % Kill TSM | CL | PA |
|---|---|---|---|---|---|---|---|
| 25 | 1000 | | 100 | | 0 | | 35 |
| | 500 | 100 | | | | 100 | |
| 26 | 1000 | | 100 | | 0 | | 45 |
| | 500 | 100 | | | | 100 | |
| 27 | 1000 | | 100 | | 40 | | 35 |
| | 500 | 100 | | | | 100 | |
| 34 | 500 | | | | | | 25 |
| | 100 | 35 | 70 | | 16 | 10 | |
| 35 | 500 | | | | 99[a] | | 90 |
| | 100 | 55 | 100 | | | 0 | |
| 36 | 500 | | | | 100 | | 60 |
| | 100 | 55 | 100 | | | 45 | |
| 37 | 1000 | | 100 | | 100 | 100 | 100 |
| 38 | 1000 | | 100 | | 100 | 100 | 100 |
| 39 | 1000 | | 100 | | 100 | 95 | 90 |
| 40 | 1000 | 100 | 100 | | 40 | | 80 |
| | 500 | | | | | 75 | |
| 41 | 1000 | | 100 | 100 | 0 | | 100 |
| | 500 | 95 | | | | 85 | |

Table 4 (continued)

| Cmpd No. | Rate (ppm) | % Kill BAW | MBB | SAW | TSM | CL | PA |
|---|---|---|---|---|---|---|---|
| 42 | 1000 | | 75 | 100 | 0 | | 100 |
| | 500 | 100 | | | | 100 | |
| 43 | 500 | 100 | 100 | | 0 | 100 | 0 |
| 44 | 500 | 100 | 100 | | 0 | 100 | 60 |
| 45 | 1000 | | | | 100 | | |
| | 500 | 100 | 100 | | | 100 | 90 |
| 46 | 1000 | | 80 | | 100 | 95 | 90 |
| 47 | 1000 | | 70 | | 100 | 100 | 100 |
| 48 | 1000 | | 95 | | 100 | 100 | 100 |
| 49 | 1000 | | 100 | | 100 | 100 | 100 |
| 50 | 1000 | | 100 | | 100 | 100 | 100 |
| 51 | 1000 | | 100 | | 100 | 100 | 100 |
| 52 | 1000 | | | | | | 0 |
| | 250 | | 75 | | | 85 | |
| 53 | 1000 | | | | | | 55 |
| | 250 | | 100 | | | 100 | |

Table 4 (continued)

| Cmpd No. | Rate (ppm) | % Kill BAW | MBB | SAW | TSM | CL | PA |
|---|---|---|---|---|---|---|---|
| 54 | 1000 | | | | | | 65 |
| | 250 | | 90 | | | 100 | |
| 55 | 1000 | | 100 | | 100 | 100 | 75 |
| 56 | 1000 | | 100 | | 100 | 100 | 95 |
| 57 | 1000 | | 100 | | 100 | 100 | 90 |
| 58 | 1000 | | 75 | | 100 | 100 | 0 |
| 59 | 1000 | | 100 | | 100 | 100 | 90 |
| 60 | 1000 | | 100 | | 100 | 100 | 80 |
| 61 | 1000 | | 60 | | 94 | 100 | 0 |
| 62 | 1000 | | 100 | | 100 | 80 | 80 |
| 63 | 1000 | | 100 | | 100 | 100 | 95 |
| 64 | 500 | | | | 23 | | |
| | 250 | | 85 | | | 100 | 80 |
| 65 | 500 | | | | 1 | | |
| | 250 | | 90 | | | 100 | 70 |
| 66 | 250 | | 100 | | 11 | 100 | 65 |
| 70 | 500 | 100 | 80 | | 1 | 40 | 0 |
| 77 | 500 | | | | 14 | | 0 |
| | 100 | 0 | 0 | | | 0 | |

Table 4 (continued)

| Cmpd No. | Rate (ppm) | % Kill BAW | MBB | SAW | TSM | CL | PA |
|---|---|---|---|---|---|---|---|
| 78 | 500 | | | | 11 | | 0 |
| | 100 | 20 | 0 | | | 0 | |
| 79 | 500 | | | | 10 | | 0 |
| | 100 | 45 | 15 | | | 0 | |
| 80 | 1000 | | 95 | | 96 | 100 | 100 |
| 84 | 1000 | | 95 | | 21 | 90 | 65 |
| 85 | 1000 | | 100 | | 14 | 100 | 95 |
| 86 | 1000 | | 80 | | 92 | 100 | 85 |
| 87 | 1000 | | 100 | | 97 | 100 | 90 |
| 88 | 1000 | | 100 | | 100 | 100 | 80 |
| 89 | 1000 | | 100 | | 100 | 100 | 100 |
| 94 | 1000 | | 100 | | 63 | 100 | 35 |
| 95 | 1000 | | 100 | | 50 | 100 | 55 |
| 96 | 1000 | | 100 | | 100 | 100 | 40 |
| 97 | 1000 | | 100 | | 99 | 100 | 100 |
| 98 | 1000 | | 100 | | 100 | 100 | 100 |
| 99 | 1000 | | 100 | | 100 | 100 | 100 |

Table 4 (continued)

| Cmpd No. | Rate (ppm) | % Kill BAW | MBB | SAW | TSM | CL | PA |
|---|---|---|---|---|---|---|---|
| 103 | 1000 | | 95 | | 100 | 85 | 95 |
| 104 | 1000 | | 100 | | 89 | 100 | 60 |
| 105 | 1000 | | 100 | | 100 | 100 | 95 |
| 109 | 1000 | | 100 | | 100 | 100 | 80 |
| 110 | 1000 | | 100 | | 100 | 100 | 100 |
| 111 | 1000 | | 100 | | 100 | 100 | 100 |
| A4 | 1000 | | 63[a] | | 0 | 5 | 0 |
| A6 | 1000 | | 68[a] | | 0 | 25 | 0 |
| A8 | 1000 | | 0 | | 0 | 0 | 0 |
| A9 | 1000 | | 0 | | 0 | 0 | 0 |
| A10 | 1000 | | 0 | | 0 | 30 | 0 |
| A11 | 1000 | | 68[a] | | 0 | 88[a] | 0 |
| A12 | 1000 | | 78[a] | | 0 | 68[a] | 0 |
| A13 | 1000 | | 95[a] | | 0 | 100[a] | 0 |
| A15 | 1000 | | 55 | | 0 | 60 | 0 |
| B7 | 1000 | | 90 | | 0 | | 0 |
| | 500 | 95 | | | | 30 | |

Table 4 (continued)

| Cmpd No. | Rate (ppm) | BAW | MBB | SAW | % Kill TSM | CL | PA |
|---|---|---|---|---|---|---|---|
| B8 | 1000 | | | | 0 | | 100 |
| | 500 | 95 | 100 | | | 100 | |
| B9 | 1000 | | 65 | | 0 | | 0 |
| | 500 | 100 | | | | 35 | |
| B12 | 500 | 100 | 100 | | 0 | 20 | 30 |
| B13 | 1000 | | 65 | | 0 | 0 | 0 |

a. Average of two tests

BAW = beet armyworm
MBB = Mexican bean beetle
SAW = southern armyworm
TSM = twospotted spider mite
CL = cabbage looper
PA = pea aphid

TABLE 5

| SOIL INSECTICIDAL TEST RESULTS | | |
|---|---|---|
| Cmpd. No. | Rate (ppm) | Initial % Kill SCR |
| 13 | 16 | 15 |
| 14 | 2 | 25 |
| 16 | 16 | 50 |
| 22 | 16 | 35 |
| 26 | 16 | 90 |
| 27 | 16 | 70 |
| 40 | 16 | 45 |
| 41 | 16 | 65 |
| 42 | 16 | 75[a] |
| 48 | 15 | 100 |
| 57 | 15 | 100 |
| 80 | 15 | 85 |
| 85 | 15 | A[b] |
| 88 | 15 | A |
| 89 | 15 | 80 |
| 94 | 15 | A |
| 95 | 15 | A |
| 96 | 15 | A |
| 99 | 15 | A |
| 103 | 15 | 85 |
| 105 | 15 | A |
| 110 | 15 | A |
| B7 | 15 | 60 |
| B8 | 15 | 60 |
| B9 | 15 | 30 |

a. = Average of two tests.
b. = A = active = >75% kill
SCR = southern corn rootworm

## Claims

1. A compound characterized by the formula

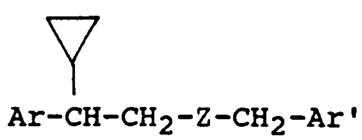

in which Ar is a thienyl or a phenyl optionally substituted with one or two groups selected from straight or branched chain ($C_{1-6}$)alkyl, halo, ($C_{1-4}$)haloalkyl, ($C_{1-4}$)alkoxy, ($C_{1-4}$)haloalkoxy, or a group having the structure -A-($CR^1R^2$)$_n$-A-, where $R^1$ and $R^2$ are independently, hydrogen, halogen, or ($C_{1-2}$)alkyl, n is 1 or 2, and each A, which may be O, S, or $CH_2$, is bonded to a carbon atom of the aromatic ring, the carbons to which the A groups are attached being adjacent to each other in the ring; Z is oxygen, sulfur, or methylene; and Ar' is 2-methyl[1,1'-biphenyl]-3-yl, 3-phenoxyphenyl, 4-fluoro-3-phenoxyphenyl, and 6-phenoxy-2-pyridyl, in which the phenyl, pyridyl, or phenoxy may be optionally substituted by halogen or lower alkyl.

2. A compound of claim 1 characterized in that Ar' is 3-phenoxyphenyl, 4-fluoro-3-phenoxyphenyl, 2-methyl[1,1'-biphenyl]-3-yl, or 6-phenoxy-2-pyridyl.

3. A compound of claim 2 characterized in that Ar is selected from phenyl, ($C_{1-6}$)alkylphenyl, halophenyl, ($C_{1-4}$)haloalkylphenyl, ($C_{1-4}$)alkoxyphenyl, ($C_{1-4}$)haloalkoxyphenyl, and 1,3-benzodioxol-5-yl.

4. A compound of claim 3 characterized in that Ar' is 3-phenoxyphenyl; Z is oxygen; and Ar is selected from phenyl, 4-fluorophenyl, 3-chlorophenyl, 4-chlorophenyl, 4-bromophenyl, 4-methylphenyl, 4-t-butylphenyl, 4-trifluoromethylphenyl, 4-methoxyphenyl, 4-ethoxyphenyl, 4-(2-fluoroethoxy)phenyl, 4-difluoromethoxyphenyl, 4-trifluoromethoxyphenyl and 1,3-benzodioxol-5-yl.

5. A compound of claim 4 characterized in that Ar is 4-chlorophenyl for which $[\alpha]_D^{25}$ in chloroform is negative

6. A compound of claim 4 characterized in that Ar is 4-trifluoromethylphenyl.

7. A compound of claim 4 characterized in that Ar is 4-trifluoromethoxyphenyl.

8. A compound of claim 3 characterized in that Ar' is 4-fluoro-3-phenoxyphenyl; Z is oxygen; and Ar is selected from 4-fluorophenyl, 3-chlorophenyl, 4-chlorophenyl, 4-bromophenyl, 4-methylphenyl, 4-t-butylphenyl, 4-trifluoromethylphenyl, 4-methoxyphenyl, 4-ethoxyphenyl, 4-(2-fluoroethoxy)phenyl, 4-difluoromethoxyphenyl, 4-trifluoromethoxyphenyl, and 1,3-benzodioxol-5-yl.

9. A compound of claim 8 characterized in that Ar is 4-chlorophenyl.

10. A compound of claim 9 characterized in that $[\alpha]_D^{25}$ in chloroform is negative.

11. A compound of claim 8 characterized in that Ar is 4-trifluoromethylphenyl.

12. A compound of claim 8 characterized in that Ar is 4-ethoxyphenyl.

13. A compound of claim 8 characterized in that Ar is 4-trifluoromethoxyphenyl.

14. A compound of claim 3 characterized in that Ar' is 2-methyl[1,1'-biphenyl]-3-yl; Z is oxygen; and Ar is selected from phenyl, 4-fluorophenyl, 3-chlorophenyl, 4-chlorophenyl, 4-bromophenyl, 4-methylphenyl, 4-t-butylphenyl, 4-trifluoromethylphenyl, 4-methoxyphenyl, 4-ethoxyphenyl, 4-(2-fluoroethoxy)phenyl, 4-difluoromethoxyphenyl, 4-trifluoromethoxyphenyl, and 1,3-benzodioxol-5-yl.

15. A compound of claim 14 characterized in that Ar is 4-trifluoromethoxyphenyl.

16. A compound of claim 3 characterized in that Ar is 4-chlorophenyl; Z is sulfur; and Ar' is selected from 3-phenoxyphenyl, 4-fluoro-3-phenoxyphenyl, and 2-methyl[1,1'-biphenyl]-3-yl.

17. A compound of claim 16 characterized in that Ar' is 4-fluoro-3-phenoxyphenyl.

18. A compound of claim 3 characterized in that Ar' is 6-phenoxy-2-pyridyl, Z is oxygen, and Ar is 4-chlorophenyl.

19. A compound of claim 3 characterized in that Ar' is selected from 3-phenoxyphenyl, 4-fluoro-3-phenoxyphenyl, and 2-methyl[1,1'-biphenyl]-3-yl; Z is methylene; and Ar is selected from 3-chlorophenyl, 4-chlorophenyl, 4-methylphenyl, 4-ethoxyphenyl, and 4-trifluoromethoxyphenyl.

20. A compound of claim 19 characterized in that Ar' is 3-phenoxyphenyl and Ar is 4-chlorophenyl.

21. A compound of claim 19 characterized in that Ar' is 3-phenoxyphenyl and Ar is 4-trifluoromethylphenyl.

22. A compound of claim 19 characterized in that Ar' is 4-fluoro-3-phenoxyphenyl and Ar is 4-chlorophenyl.

23. A compound of claim 19 characterized in that Ar' is 4-fluoro-3-phenoxyphenyl and Ar is 4-trifluoromethylphenyl.

**24.** A compound of claim 19 characterized in that Ar' is 4-fluoro-3-phenoxyphenyl and Ar is 4-trifluoromethoxyphenyl.

**25.** A compound of claim 19 characterized in that Ar' is 2-methyl[1,1'-biphenyl]-3-yl and Ar is 4-trifluoromethylphenyl.

**26.** A compound characterized by the formula:

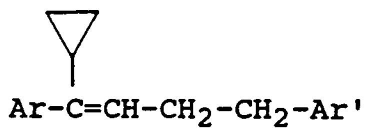

in which Ar is a thienyl or a phenyl optionally substituted with one or two groups selected from straight or branched chain ($C_{1-6}$)alkyl, halo, ($C_{1-4}$)haloalkyl, ($C_{1-4}$)alkoxy, ($C_{1-4}$)haloalkoxy, or a group having the structure -A-($CR^1R^2$)$_n$-A-, where $R^1$ and $R^2$ are independently, hydrogen, halogen, or ($C_{1-2}$)alkyl, n is 1 or 2, and each A, which may be O, S, or $CH_2$, is bonded to a carbon atom of the aromatic ring, the carbons to which the A groups are attached being adjacent to each other in the ring and Ar' is 2-methyl[1,1'-biphenyl]-3-yl, 3-phenoxyphenyl, 4-fluoro-3-phenoxyphenyl, and 6-phenoxy-2-pyridyl, in which the phenyl, pyridyl, or phenoxy may be optionally substituted by halogen or lower alkyl.

**27.** A compound of claim 26 characterized in that Ar' is selected from 3-phenoxyphenyl and 4-fluoro-3-phenoxyphenyl and Ar is selected from 4-chlorophenyl, 4-bromophenyl, and 4-methoxyphenyl.

**28.** A compound of claim 27 characterized in that Ar' is 4-fluoro-3-phenoxyphenyl and Ar is 4-chlorophenyl.

**29.** A compound characterized by the formula:

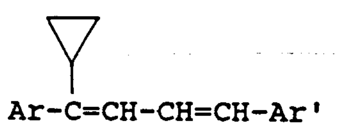

in which Ar is a thienyl or a phenyl optionally substituted with one or two groups selected from straight or branched chain ($C_{1-6}$)alkyl, halo, ($C_{1-4}$)haloalkyl, ($C_{1-4}$)alkoxy, ($C_{1-4}$)haloalkoxy, or a group having the structure -A-($CR^1R^2$)$_n$-A-, where $R^1$ and $R^2$ are independently, hydrogen, halogen, or ($C_{1-2}$)alkyl, n is 1 or 2, and each A, which may be O, S, or $CH_2$, is bonded to a carbon atom of the aromatic ring, the carbons to which the A groups are attached being adjacent to each other in the ring and Ar' is 2-methyl[1,1'-biphenyl]-3-yl, 3-phenoxyphenyl, 4-fluoro-3-phenoxyphenyl, and 6-phenoxy-2-pyridyl, in which the phenyl, pyridyl, or phenoxy may be optionally substituted by halogen or lower alkyl.

**30.** A compound of claim 29 characterized in that Ar' is selected from 3-phenoxyphenyl, 4-fluoro-3-phenoxyphenyl, and 2-methyl[1,1'-biphenyl]-3-yl and Ar is selected from 4-chlorophenyl, 4-methylphenyl, 4-trifluoromethylphenyl, and 4-ethoxyphenyl.

**31.** An insecticidal or acaricidal composition characterized by an insecticidally or acaricidally effective amount of a compound of claim 1 in admixture with one or more compatible agricultural carriers, diluents, adjuvants, or complementary pesticides.

**32.** An insecticidal composition characterized by an insecticidally effective amount of a compound of claim 26 in admixture with one or more compatible agricultural carriers, diluents, adjuvants, or complementary pesticides.

**33.** An insecticidal composition characterized by an insecticidally effective amount of a compound of claim 29 in admixture with one or more compatible agricultural carriers, diluents, adjuvants, or complementary pesticides.

**34.** A method of controlling insects and acarids characterized by applying to the locus where control is desired an insecticidally or acaricidally effective amount of a compound of claim 1.

**35.** A method of controlling insects characterized by applying to the locus where control is desired an insecticidally effective amount of a compound of claim 26.

**36.** A method of controlling insects characterized by applying to the locus where control is desired an insecticidally effective amount of a compound of claim 29.

**37.** A process for preparing a compound of the formula

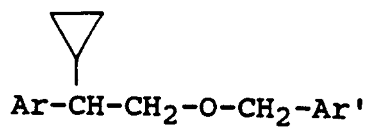

in which Ar is a thienyl or a phenyl optionally substituted with one or two groups selected from straight or branched chain $(C_{1-6})$alkyl, halo, $(C_{1-4})$haloalkyl, $(C_{1-4})$alkoxy, $(C_{1-4})$haloalkoxy, or a group having the structure $-A-(CR^1R^2)_n-A-$, where $R^1$ and $R^2$ are independently, hydrogen, halogen, or $(C_{1-2})$alkyl, n is 1 or 2, and each A, which may be O, S, or $CH_2$, is bonded to a carbon atom of the aromatic ring, the carbons to which the A groups are attached being adjacent to each other in the ring and Ar' is 2-methyl[1,1'-biphenyl]-3-yl, 3-phenoxyphenyl, 4-fluoro-3-phenoxyphenyl, and 6-phenoxy-2-pyridyl, in which the phenyl, pyridyl, or phenoxy may be optionally substituted by halogen or lower alkyl, characterized in that a compound of the formula

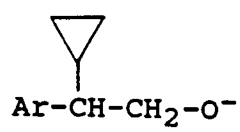

is reacted with a compound of the formula,

$Ar'-CH_2-X$

wherein X is a leaving group capable of being displaced by ethoxide ions.

**38.** A process for preparing a compound of the formula

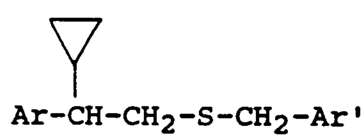

in which Ar is a phenyl optionally substituted with one or two groups selected from straight or branched chain $(C_{1-6})$alkyl, halo, $(C_{1-4})$haloalkyl, $(C_{1-4})$alkoxy, $(C_{1-4})$haloalkoxy, or a group having the structure $-A-(CR^1R^2)_n-A-$, where $R^1$ and $R^2$ are independently, hydrogen, halogen, or $(C_{1-2})$alkyl, n is 1 or 2, and each A, which may be O, S, or $CH_2$, is bonded to a carbon atom of the aromatic ring, the carbons to which the A groups are attached being adjacent to each other in the ring and Ar' is 2-methyl[1,1'-biphenyl]-3-yl, 3-phenoxyphenyl, 4-fluoro-3-phenoxyphenyl, and 6-phenoxy-2-pyridyl, in which the phenyl, pyridyl, or phenoxy may be optionally substituted by halogen or lower alkyl, characterized in that a compound of the formula

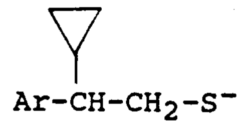

is reacted with a compound of the formula

$Ar'-CH_2-X$

wherein X is a leaving group capable of being displaced by thioethoxide ions.

**39.** A process for preparing a compound of the formula

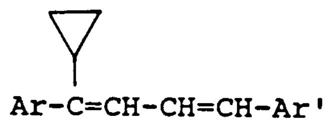

in which Ar is a phenyl optionally substituted with one or two groups selected from straight or branched chain $(C_{1-6})$alkyl, halo, $(C_{1-4})$haloalkyl, $(C_{1-4})$alkoxy, $(C_{1-4})$haloalkoxy, or a group having the structure $-A-(CR^1R^2)_n-A-$, where $R^1$ and $R^2$ are independently, hydrogen, halogen, or $(C_{1-2})$alkyl, n is 1 or 2, and each A, which may be O, S, or $CH_2$, is bonded to a carbon atom of the aromatic ring, the carbons to which the A groups are attached being adjacent to each other in the ring and Ar' is 2-methyl[1,1'-biphenyl]-3-yl, 3-phenoxyphenyl, 4-fluoro-3-phenoxyphenyl, and 6-phenoxy-2-pyridyl, in which the phenyl, pyridyl, or phenoxy may be optionally substituted by halogen or lower alkyl, characterized in that a compound of the formula

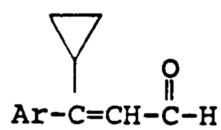

is reacted with a compound of the formula

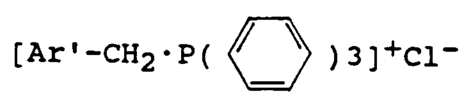

**40.** A process for preparing a compound of the formula

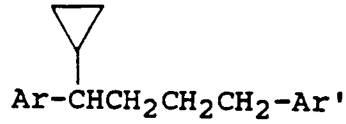

in which Ar is a thienyl or a phenyl optionally substituted with one or two groups selected from straight or branched chain $(C_{1-6})$alkyl, halo, $(C_{1-4})$haloalkyl, $(C_{1-4})$alkoxy, $(C_{1-4})$haloalkoxy, or a group having the structure $-A-(CR^1R^2)_n-A-$, where $R^1$ and $R^2$ are independently, hydrogen, halogen, or $(C_{1-2})$alkyl, n is 1 or 2, and each A, which may be O, S, or $CH_2$, is bonded to a carbon atom of the aromatic ring, the carbons to which the A groups are attached being adjacent to each other in the ring and Ar' is 2-methyl[1,1'-biphenyl]-3-yl, 3-phenoxyphenyl, 4-fluoro-3-phenoxyphenyl, and 6-phenoxy-2-pyridyl, in which the phenyl, pyridyl, or phenoxy may be optionally substituted by halogen or lower alkyl,

characterized in that a compound of the formula

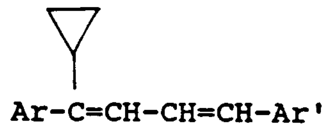

is hydrogenated.

**41.** A process for preparing a compound of the formula

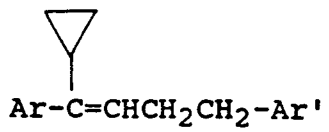

wherein Ar is a phenyl optionally substituted with one or two groups selected from straight or branched chain $(C_{1-6})$alkyl, halo, $(C_{1-4})$haloalkyl, $(C_{1-4})$alkoxy, $(C_{1-4})$haloalkoxy, or a group having the structure $-A-(CR^1R^2)_n-A-$, where $R^1$ and $R^2$ are independently, hydrogen, halogen, or $(C_{1-2})$alkyl, n is 1 or 2, and each A, which may be O, S, or $CH_2$, is bonded to a carbon atom of the aromatic ring, the carbons to which the A groups are attached being adjacent to each other in the ring and Ar' is 2-methyl[1,1'-biphenyl]-3-yl, 3-phenoxyphenyl, 4-fluoro-3-phenoxyphenyl, and 6-phenoxy-2-pyridyl, in which the phenyl, pyridyl, or phenoxy may be optionally substituted by halogen or lower alkyl, characterized in that a compound of the formula

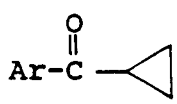

is reacted with a compound of the formula

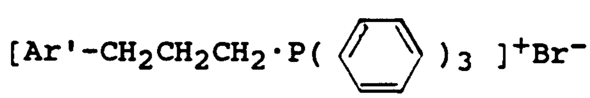

**42.** A process for preparing a compound of the formula

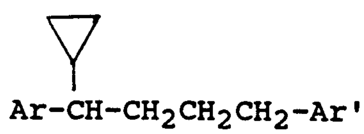

in which Ar is a phenyl optionally substituted with one or two groups selected from straight or branched chain $(C_{1-6})$alkyl, halo, $(C_{1-4})$haloalkyl, $(C_{1-4})$alkoxy, $(C_{1-4})$haloalkoxy, or a group having the structure $-A-(CR^1R^2)_n-A-$, where $R^1$ and $R^2$ are independently, hydrogen, halogen, or $(C_{1-2})$alkyl, n is 1 or 2, and each A, which may be O, S, or $CH_2$, is bonded to a carbon atom of the aromatic ring, the carbons to which the A groups are attached being adjacent to each other in the ring and Ar' is 2-methyl[1,1'-biphenyl]-3-yl, 3-phenoxyphenyl, 4-fluoro-3-phenoxyphenyl, and 6-phenoxy-2-pyridyl, in which the phenyl, pyridyl, or phenoxy may be optionally substituted by halogen or lower alkyl, characterized in that a compound of the formula

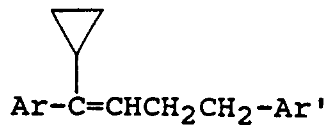

is hydrogenated.

## Patentansprüche

1. Verbindung, gekennzeichnet durch die Formel

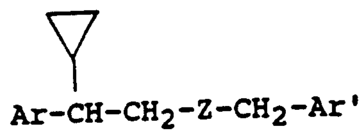

in der Ar eine Thienyl- oder eine gegebenenfalls mit einem oder zwei Resten substituierte Phenylgruppe bedeutet, wobei die Reste ausgewählt sind aus geradkettigen oder verzweigten $C_{1-6}$-Alkylresten, Halogenatomen, $C_{1-4}$-Haloalkyl-, $C_{1-4}$-Alkoxy-, $C_{1-4}$-Haloalkoxyresten oder einem Rest mit der Struktur -A-$(CR^1R^2)_n$-A-, wobei $R^1$ und $R^2$ unabhängig voneinander ein Wasserstoffatom, ein Halogenatom oder einen $C_{1-2}$-Alkylrest bedeuten, n den Wert 1 oder 2 hat und jeder Rest A, der O, S oder $CH_2$ bedeuten kann, an ein Kohlenstoffatom des aromatischen Rings gebunden ist, wobei die Kohlenstoffatome, an die die Reste A gebunden sind, sich nebeneinander im Ring befinden; Z ein Sauerstoffatom, Schwefelatom oder eine Methylengruppe bedeutet; und Ar' eine 2-Methyl[1,1'-biphenyl]-3-yl-, 3-Phenoxyphenyl-, 4-Fluor-3-phenoxyphenyl- und 6-Phenoxy-2-pyridylgruppe bedeutet, wobei die Phenyl-, Pyridyl- oder Phenoxygruppen gegebenenfalls durch Halogenatome oder Niederalkylreste substituiert sein können.

2. Verbindung nach Anspruch 1, dadurch gekennzeichnet, daß Ar' eine 3-Phenoxyphenyl-, 4-Fluor-3-phenoxyphenyl-, 2-Methyl-[1,1'-biphenyl]-3-yl- oder 6-Phenoxy-2-pyridylgruppe bedeutet.

3. Verbindung nach Anspruch 2, dadurch gekennzeichnet, daß Ar eine Phenyl-, $C_{1-6}$-Alkylphenyl-, Halophenyl-, $C_{1-4}$-Haloalkylphenyl-, $C_{1-4}$-Alkoxyphenyl-, $C_{1-4}$-Haloalkoxyphenyl- oder eine 1,3-Benzodioxol-5-yl-Gruppe bedeutet.

4. Verbindung nach Anspruch 3, dadurch gekennzeichnet, daß Ar' eine 3-Phenoxyphenylgruppe bedeutet; Z ein Sauerstoffatom darstellt; und Ar ausgewählt ist aus einer Phenyl-, 4-Fluorphenyl-, 3-Chlorphenyl-, 4-Chlorphenyl-, 4-Bromphenyl-, 4-Methylphenyl-, 4-tert.-Butylphenyl-, 4-Trifluormethylphenyl-, 4-Methoxyphenyl-, 4-Ethoxyphenyl-, 4-(2-Fluorethoxy)phenyl-, 4-Difluormethoxyphenyl-, 4-Trifluormethoxyphenyl- und einer 1,3-Benzodioxol-5-yl-Gruppe.

5. Verbindung nach Anspruch 4, dadurch gekennzeichnet, daß Ar eine 4-Chlorphenylgruppe bedeutet, für die $[\alpha]_D^{25}$ in Chloroform negativ ist.

6. Verbindung nach Anspruch 4, dadurch gekennzeichnet, daß Ar eine 4-Trifluormethylphenylgruppe ist.

7. Verbindung nach Anspruch 4, dadurch gekennzeichnet, daß Ar eine 4-Trifluormethoxyphenylgruppe ist.

8. Verbindung nach Anspruch 3, dadurch gekennzeichnet, daß Ar' eine 4-Fluor-3-phenoxyphenylgruppe bedeutet, Z ein Sauerstoffatom ist, und Ar ausgewählt ist aus einer 4-Fluorphenyl-, 3-Chlorphenyl-, 4-Chlorphenyl-, 4-Bromphenyl-, 4-Methylphenyl-, 4-tert.-Butylphenyl-, 4-Trifluormethylphenyl-, 4-Methoxyphenyl-, 4-Ethoxyphenyl-, 4-(2-Fluorethoxy)-phenyl-, 4-Difluormethoxyphenyl-, 4-Trifluormethoxyphenyl- und 1,3-Benzodioxol-5-yl-Gruppe.

9. Verbindung nach Anspruch 8, dadurch gekennzeichnet, daß Ar eine 4-Chlorphenylgruppe ist.

**10.** Verbindung nach Anspruch 9, dadurch gekennzeichnet, daß $[\alpha]_D^{25}$ in Chloroform negativ ist.

**11.** Verbindung nach Anspruch 8, dadurch gekennzeichnet, daß Ar eine 4-Trifluormethylphenylgruppe ist.

**12.** Verbindung nach Anspruch 8, dadurch gekennzeichnet, daß Ar eine 4-Ethoxyphenylgruppe ist.

**13.** Verbindung nach Anspruch 8, dadurch gekennzeichnet, daß Ar eine 4-Trifluormethoxyphenylgruppe ist.

**14.** Verbindung nach Anspruch 3, dadurch gekennzeichnet, daß Ar' eine 2-Methyl[1,1'-biphenyl]-3-yl-Gruppe ist; Z ein Sauerstoffatom darstellt; und Ar ausgewählt ist aus einer Phenyl-, 4-Fluorphenyl-, 3-Chlorphenyl-, 4-Chlorphenyl-, 4-Bromphenyl-, 4-Methylphenyl-, 4-tert.-Butylphenyl-, 4-Trifluormethylphenyl-, 4-Methoxyphenyl-, 4-Ethoxyphenyl-, 4-(2-Fluorethoxy)phenyl-, 4-Difluormethoxyphenyl-, 4-Trifluormethoxyphenyl- und 1,3-Benzodioxol-5-yl-Gruppe.

**15.** Verbindung nach Anspruch 14, dadurch gekennzeichnet, daß Ar eine 4-Trifluormethoxyphenylgruppe ist.

**16.** Verbindung nach Anspruch 3, dadurch gekennzeichnet, daß Ar eine 4-Chlorphenylgruppe ist; Z ein Schwefelatom darstellt; und Ar' ausgewählt ist aus einer 3-Phenoxyphenyl-, 4-Fluor-3-phenoxyphenyl- und 2-Methyl[1,1'-biphenyl]-3-yl-Gruppe.

**17.** Verbindung nach Anspruch 16, dadurch gekennzeichnet, daß Ar' eine 4-Fluor-3-phenoxyphenylgruppe ist.

**18.** Verbindung nach Anspruch 3, dadurch gekennzeichnet, daß Ar' eine 6-Phenoxy-2-pyridylgruppe ist, Z ein Sauerstoffatom darstellt und Ar eine 4-Chlorphenylgruppe ist.

**19.** Verbindung nach Anspruch 3, dadurch gekennzeichnet, daß Ar' ausgewählt ist aus einer 3-Phenoxyphenyl-, 4-Fluor-3-phenoxyphenyl- und 2-Methyl[1,1'-biphenyl]-3-yl-Gruppe, Z eine Methylengruppe darstellt und Ar ausgewählt ist aus einer 3-Chlorphenyl-, 4-Chlorphenyl-, 4-Methylphenyl-, 4-Ethoxyphenyl- und 4-Trifluormethoxyphenylgruppe.

**20.** Verbindung nach Anspruch 19, dadurch gekennzeichnet, daß Ar' eine 3-Phenoxyphenylgruppe und Ar eine 4-Chlorphenylgruppe ist.

**21.** Verbindung nach Anspruch 19, dadurch gekennzeichnet, daß Ar' eine 3-Phenoxyphenylgruppe und Ar eine 4-Trifluormethylphenylgruppe ist.

**22.** Verbindung nach Anspruch 19, dadurch gekennzeichnet, daß Ar' eine 4-Fluor-3-phenoxyphenylgruppe und Ar eine 4-Chlorphenylgruppe ist.

**23.** Verbindung nach Anspruch 19, dadurch gekennzeichnet, daß Ar' eine 4-Fluor-3-phenoxyphenylgruppe und Ar eine 4-Trifluormethylphenylgruppe ist.

**24.** Verbindung nach Anspruch 19, dadurch gekennzeichnet, daß Ar' eine 4-Fluor-3-phenoxyphenylgruppe und Ar eine 4-Trifluormethoxyphenylgruppe ist.

**25.** Verbindung nach Anspruch 19, dadurch gekennzeichnet, daß Ar' eine 2-Methyl[1,1'-biphenyl]-3-yl-Gruppe und Ar eine 4-Trifluormethylphenylgruppe ist.

**26.** Verbindung, gekennzeichnet durch die Formel

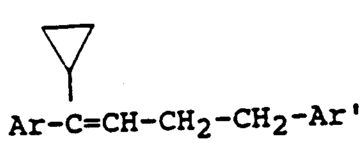

in der Ar eine Thienylgruppe oder eine gegebenenfalls mit einem oder zwei Resten substituierte Phenylgruppe darstellt, wobei die Reste ausgewählt sind aus geradkettigen oder verzweigten $C_{1-6}$-Alkylresten, Halogenatomen, $C_{1-4}$-Haloalkyl-, $C_{1-4}$-Alkoxy-, $C_{1-4}$-Haloalkoxyresten oder einem Rest mit der Struktur -A-$(CR^1R^2)_n$-A-, wobei $R^1$ und $R^2$ unabhängig voneinander ein Wasserstoffatom, ein Halogenatom, oder einen $C_{1-2}$-Alkylrest bedeuten, n den Wert 1 oder 2 hat und jeder Rest A, der O, S oder $CH_2$ bedeuten kann, an ein Kohlenstoffatom des aromatischen Rings gebunden ist, wobei die Kohlenstoffatome, an die die Reste A gebunden sind, sich nebeneinander im Ring befinden, und Ar' eine 2-Methyl[1,1'-biphenyl]-3-yl-, 3-Phenoxyphenyl-, 4-Fluor-3-phenoxyphenyl- und 6-Phenoxy-2-pyridylgruppe bedeutet, wobei die Phenyl-, Pyridyl- oder Phenoxygruppen gegebenenfalls durch Halogenatome oder Niederalkylreste substituiert sein können.

**27.** Verbindung nach Anspruch 26, dadurch gekennzeichnet, daß Ar' ausgewählt ist aus einer 3-Phenoxyphenyl- und 4-Fluor-3-phenoxyphenylgruppe und Ar ausgewählt ist aus einer 4-Chlorphenyl-, 4-Bromphenyl- und 4-Methoxyphenylgruppe.

**28.** Verbindung nach Anspruch 27, dadurch gekennzeichnet, daß Ar' eine 4-Fluor-3-phenoxyphenylgruppe und Ar eine 4-Chlorphenylgruppe ist.

**29.** Verbindung, gekennzeichnet durch die Formel

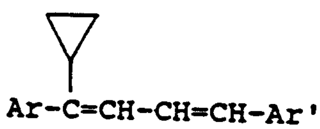

in der Ar eine Thienylgruppe oder eine gegebenenfalls mit einem oder zwei Resten substituierte Phenylgruppe bedeutet, wobei die Reste ausgewählt sind aus geradkettigen oder verzweigten $C_{1-6}$-Alkylresten, Halogenatomen, $C_{1-4}$-Haloalkyl-, $C_{1-4}$-Alkoxy-, $C_{1-4}$-Haloalkoxyresten oder einem Rest mit der Struktur -A-$(CR^1R^2)_n$-A-, wobei $R^1$ und $R^2$ unabhängig voneinander ein Wasserstoffatom, ein Halogenatom oder einen $C_{1-2}$-Alkylrest bedeuten, n den Wert 1 oder 2 hat und jeder Rest A, der O, S oder $CH_2$ bedeuten kann, an ein Kohlenstoffatom des aromatischen Rings gebunden ist, wobei die Kohlenstoffatome, an die die Reste A gebunden sind, sich nebeneinander im Ring befinden, und Ar' eine 2-Methyl[1,1'-biphenyl]-3-yl-, 3-Phenoxyphenyl-, 4-Fluor-3-phenoxyphenyl- und 6-Phenoxy-2-pyridylgruppe bedeutet, wobei die Phenyl-, Pyridyl- oder Phenoxygruppen gegebenenfalls durch Halogenatome oder Niederalkylreste substituiert sein können.

**30.** Verbindung nach Anspruch 29, dadurch gekennzeichnet, daß Ar' ausgewählt ist aus einer 3-Phenoxyphenyl-, 4-Fluor-3-phenoxyphenyl- und 2-Methyl-[1,1'-biphenyl]-3-yl-Gruppe und Ar ausgewählt ist aus einer 4-Chlorphenyl-, 4-Methylphenyl-, 4-Trifluormethylphenyl- und 4-Ethoxyphenylgruppe.

**31.** Insektizides oder akarizides Mittel, gekennzeichnet durch eine insektizid oder akarizid wirksame Menge einer Verbindung nach Anspruch 1 im Gemisch mit einem oder mehreren verträglichen landwirtschaftlichen Trägern, Verdünnungsmitteln, Hilfsmitteln oder ergänzenden Pestiziden.

**32.** Insektizides Mittel, gekennzeichnet durch eine insektizid wirksame Menge einer Verbindung nach Anspruch 26 im Gemisch mit einem oder mehreren verträglichen landwirtschaftlichen Trägern, Verdünnungsmitteln, Hilfsmitteln oder ergänzenden Pestiziden.

**33.** Insektizides Mittel, gekennzeichnet durch eine insektizid wirksame Menge einer Verbindung nach Anspruch 29 im Gemisch mit einem oder mehreren verträglichen landwirtschaftlichen Trägern, Verdünnungsmitteln, Hilfsmitteln oder ergänzenden Pestiziden.

**34.** Verfahren zur Bekämpfung von Insekten und Akariden, gekennzeichnet durch das Aufbringen einer insektizid oder akarizid wirksamen Menge einer Verbindung nach Anspruch 1 an dem Ort, wo die Bekämpfung erwünscht ist.

**35.** Verfahren zur Bekämpfung von Insekten, gekennzeichnet durch das Aufbringen einer insektizid wirksamen Menge einer Verbindung nach Anspruch 26 an dem Ort, wo die Bekämpfung erwünscht ist.

**36.** Verfahren zur Bekämpfung von Insekten, gekennzeichnet durch das Aufbringen einer insektizid wirksamen Menge einer Verbindung nach Anspruch 29 an dem Ort, wo die Bekämpfung erwünscht ist.

**37.** Verfahren zur Herstellung einer Verbindung der Formel

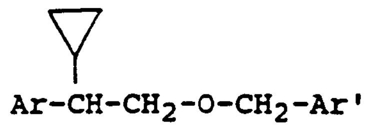

in der Ar eine Thienylgruppe oder eine gegenenfalls mit einem oder zwei Resten substituierte Phenylgruppe bedeutet, wobei die Reste ausgewählt sind aus geradkettigen oder verzweigten $C_{1-6}$-Alkylresten, Halogenatomen, $C_{1-4}$-Haloalkyl-, $C_{1-4}$-Alkoxy-, $C_{1-4}$-Haloalkoxyresten oder einem Rest mit der Struktur $-A-(CR^1R^2)_n-A-$, wobei $R^1$ und $R^2$ unabhängig voneinander ein Wasserstoffatom, ein Halogenatom oder einen $C_{1-2}$-Alkylrest bedeuten, n den Wert 1 oder 2 hat und jeder Rest A, der O, S oder $CH_2$ bedeuten kann, an ein Kohlenstoffatom des aromatischen Rings gebunden ist, wobei die Kohlenstoffatome, an die die Reste A gebunden sind, sich nebeneinander im Ring befinden, und Ar' eine 2-Methyl[1,1'-biphenyl)-3-yl-, 3-Phenoxyphenyl-, 4-Fluor-3-phenoxyphenyl- und 6-Phenoxy-2-pyridylgruppe bedeutet, wobei die Phenyl-, Pyridyl- oder Phenoxygruppen gegenenfalls durch Halogenatome oder Niederalkylreste substituiert sein können, dadurch gekennzeichnet, daß eine Verbindung der Formel

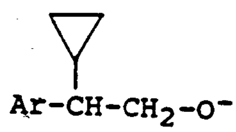

mit einer Verbindung der Formel

$Ar'-CH_2-X$

in der X eine Abgangsgruppe ist, die durch Ethoxidionen ersetzt werden kann, umgesetzt wird.

**38.** Verfahren zur Herstellung einer Verbindung der Formel

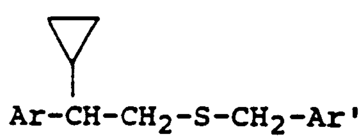

in der Ar eine gegebenenfalls mit einem oder zwei Resten substituierte Phenylgruppe ist, wobei die Reste ausgewählt sind aus geradkettigen oder verzweigten $C_{1-6}$-Alkylresten, Halogenatomen, $C_{1-4}$-Haloalkyl-, $C_{1-4}$-Alkoxy-, $C_{1-4}$-Haloalkoxyresten oder einem Rest mit der Struktur $-A-(CR^1R^2)_n-A-$, wobei $R^1$ und $R^2$ unabhängig voneinander ein Wasserstoffatom, ein Halogenatom oder einen $C_{1-2}$-Alkylrest bedeuten, n den Wert 1 oder 2 hat und jeder Rest A, der O, S oder $CH_2$ bedeuten kann, an ein Kohlenstoffatom des aromatischen Rings gebunden ist, wobei die Kohlenstoffatome, an die die Reste A gebunden sind, sich nebeneinander im Ring befinden, und Ar' eine 2-Methyl[1,1'-biphenyl]-3-yl-,3-Phenoxyphenyl-, 4-Fluor-3-phenoxyphenyl- und 6-Phenoxy-2-pyridylgruppe bedeutet, in der die Phenyl-, Pyridyl- oder Phenoxygruppen gegebenenfalls durch Halogenatome oder Niederalkylreste substituiert sein können, dadurch gekennzeichnet, daß eine Verbindung der Formel

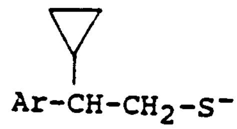

mit einer Verbindung der Formel

Ar'-$CH_2$-X

in der X eine Abgangsgruppe ist, die durch Thioethoxidionen ersetzt werden kann, umgesetzt wird.

**39.** Verfahren zur Herstellung einer Verbindung der Formel

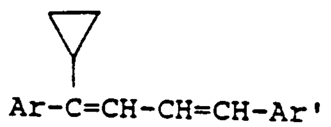

in der Ar eine gegebenenfalls mit einem oder zwei Resten substituierte Phenylgruppe bedeutet, wobei die Reste ausgewählt sind aus geradkettigen oder verzweigten $C_{1-6}$-Alkylresten, Halogenatomen, $C_{1-4}$-Haloalkyl-, $C_{1-4}$-Alkoxy-, $C_{1-4}$-Haloalkoxyresten oder einem Rest mit der Struktur -A-$(CR^1R^2)_n$-A-, wobei $R^1$ und $R^2$ unabhängig voneinander ein Wasserstoffatom, ein Halogenatom oder einen $C_{1-2}$-Alkylrest bedeuten, n den Wert 1 oder 2 hat und jeder Rest A, der O, S oder $CH_2$ bedeuten kann, an ein Kohlenstoffatom des aromatischen Rings gebunden ist, wobei die Kohlenstoffatome, an die die Reste A gebunden sind, sich nebeneinander im Ring befinden, und Ar' eine 2-Methyl-[1,1'-biphenyl]-3-yl-, 3-Phenoxyphenyl-, 4-Fluor-3-phenoxyphenyl- und 6-Phenoxy-2-pyridylgruppe bedeutet, wobei die Phenyl-, Pyridyl- oder Phenoxygruppen gegebenenfalls durch Halogenatome oder Niederalkylreste substituiert sein können, dadurch gekennzeichnet, daß eine Verbindung der Formel

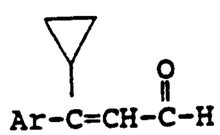

mit einer Verbindung der Formel

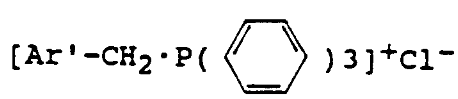

umgesetzt wird.

**40.** Verfahren zur Herstellung einer Verbindung der Formel

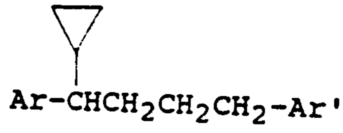

in der Ar eine Thienylgruppe oder eine gegebenenfalls mit einem oder zwei Resten substituierte Phenylgruppe bedeutet, wobei die Reste ausgewählt sind aus geradkettigen oder verzweigten $C_{1-6}$-Alkylresten, Halogenatomen, $C_{1-4}$-Haloalkyl-, $C_{1-4}$-Alkoxy-, $C_{1-4}$-Haloalkoxyresten oder einem Rest mit der Struktur -A-$(CR^1R^2)_n$-A-, wobei $R^1$ und $R^2$ unabhängig voneinander ein Wasserstoffatom, ein

Halogenatom oder einen $C_{1-2}$-Alkylrest bedeuten, n den Wert 1 oder 2 hat und jeder Rest A, der O, S oder $CH_2$ bedeuten kann, an ein Kohlenstoffatom des aromatischen Rings gebunden ist, wobei die Kohlenstoffatome, an die die Reste A gebunden sind, sich nebeneinander im Ring befinden, und Ar' eine 2-Methyl[1,1'-biphenyl]-3-yl-, 3-Phenoxyphenyl-, 4-Fluor-3-phenoxyphenyl- und 6-Phenoxy-2-pyridylgruppe bedeutet, wobei die Phenyl-, Pyridyl- oder Phenoxygruppen gegebenenfalls durch Halogenatome oder Niederalkylreste substituiert sein können, dadurch gekennzeichnet, daß eine Verbindung der Formel

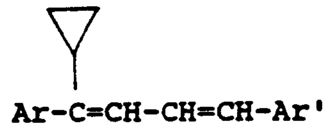

hydriert wird.

**41.** Verfahren zur Herstellung einer Verbindung der Formel

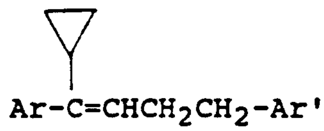

in der Ar eine gegebenenfalls mit einem oder zwei Resten substituierte Phenylgruppe bedeutet, wobei die Reste ausgewählt sind aus geradkettigen oder verzweigten $C_{1-6}$-Alkylresten, Halogenatomen, $C_{1-4}$-Haloalkyl-, $C_{1-4}$-Alkoxy-, $C_{1-4}$-Haloalkoxyresten oder einem Rest mit der Struktur $-A-(CR^1R^2)_n-A-$, wobei $R^1$ und $R^2$ unabhängig voneinander ein Wasserstoffatom, ein Halogenatom oder einen $C_{1-2}$-Alkylrest bedeuten, n den Wert 1 oder 2 hat und jeder Rest A, der O, S oder $CH_2$ bedeuten kann, an ein Kohlenstoffatom des aromatischen Rings gebunden ist, wobei die Kohlenstoffatome, an die die Reste A gebunden sind, sich nebeneinander im Ring befinden, und Ar' eine 2-Methyl[1,1'-biphenyl]-3-yl-, 3-Phenoxyphenyl-, 4-Fluor-3-phenoxyphenyl- und 6-Phenoxy-2-pyridylgruppe bedeutet, wobei die Phenyl-, Pyridyl- oder Phenoxygruppen gegebenenfalls durch Halogenatome oder Niederalkylreste substituiert sein können, dadurch gekennzeichnet, daß eine Verbindung der Formel

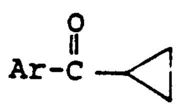

mit einer Verbindung der Formel

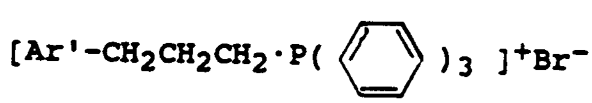

umgesetzt wird.

**42.** Verfahren zur Herstellung einer Verbindung der Formel

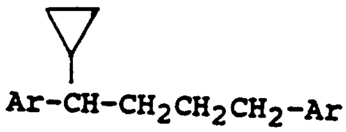

in der Ar eine gegebenenfalls mit einem oder zwei Resten substituierte Phenylgruppe bedeutet, wobei die Reste ausgewählt sind aus geradkettigen oder verzweigten $C_{1-6}$-Alkylresten, Halogenatomen, $C_{1-4}$-Haloalkyl-, $C_{1-4}$-Alkoxy-, $C_{1-4}$-Haloalkoxyresten oder einem Rest mit der Struktur $-A-(CR^1R^2)_n-A-$, wobei $R^1$ und $R^2$ unabhängig voneinander ein Wasserstoffatom, ein Halogenatom oder einen $C_{1-2}$-Alkylrest bedeuten, n den Wert 1 oder 2 hat und jeder Rest A, der O, S oder $CH_2$ bedeuten kann, an ein Kohlenstoffatom des aromatischen Rings gebunden ist, wobei die Kohlenstoffatome, an die die Reste A gebunden sind, sich nebeneinander im Ring befinden, und Ar' eine 2-Methyl[1,1'-biphenyl]-3-yl-, 3-Phenoxyphenyl-, 4-Fluor-3-phenoxyphenyl-und 6-Phenoxy-2-pyridylgruppe bedeutet, wobei die Phenyl-, Pyridyl oder Phenoxygruppen gegebenenfalls durch Halogenatome oder Niederalkylreste substituiert sein können, dadurch gekennzeichnet, daß eine Verbindung der Formel

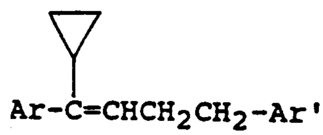

hydriert wird.

**Revendications**

1. Composé caractérisé par la formule

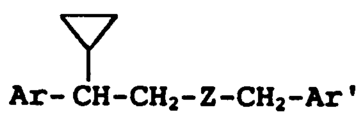

dans lequel Ar est un thiényle ou un phényle éventuellement substitué par un ou deux groupes choisis parmi un alkyle ($C_{1-6}$) à chaîne linéaire ou ramifiée, un halogène, un haloalkyle ($C_{1-4}$), un alkoxy ($C_{1-4}$), un haloalkoxy ($C_{1-4}$), ou un groupe de structure $-A-(CR^1R^2)_n-A-$, où $R^1$ et $R^2$ sont indépendamment, de l'hydrogène, un halogène, ou un alkyle ($C_{1-2}$), n est égal à 1 ou 2, et chaque A, qui peut être O, S, ou $CH_2$, est lié à un atome de carbone du cycle aromatique, les carbones auxquels sont fixés les groupes A étant adjacents les uns aux autres dans le cycle ; Z est de l'oxygène, du soufre, ou du méthylène ; et Ar' est du 2-méthyl[1,1'-biphényl]-3-yle, du 3-phénoxyphényle, du 4-fluoro-3-phénoxyphényle, et du 6-phénoxy-2-pyridyle, dans lesquels le phényle, le pyridyle, ou le phénoxy peuvent éventuellement être substitués par un halogène ou un alkyle inférieur.

2. Composé de la revendication 1 caractérisé en ce que Ar' est du 3-phénoxyphényl, du 4-fluoro-3-phénoxyphényle, du 2-méthyl[1,1'-biphényl]-3-yle, ou du 6-phénoxy-2-pyridyle.

3. Composé de la revendication 2 caractérisé en ce que Ar est choisi parmi le phényle, un alkylphényle ($C_{1-6}$), un halophényle, un haloalkylphényle ($C_{1-4}$), un alkoxyphényle ($C_{1-4}$), un haloalkoxyphényle ($C_{1-4}$), et un 1,3-benzodioxol-5-yle.

4. Composé de la revendication 3 caractérisé en ce que Ar' est du 3-phénoxyphényle ; Z est de l'oxygène ; et Ar est choisi parmi le phényle, le 4-fluorophényle, le 3-chlorophényle, le 4-chlorophényle, le 4-bromophényle, le 4-méthylphényle, le 4-t-butylphényle, le 4-trifluorométhylphényle, le 4-méthoxyphényle, le 4-éthoxyphényle, le 4-(2-fluoroéthoxy)phényle, le 4-difluorométhoxyphényle, le 4-trifluorométhoxyphényle et le 1,3-benzodioxol-5-yle.

**5.** Composé de la revendication 4 caractérisé en ce que Ar est du 4-chlorophényle pour lequel $[\alpha]_D^{25}$ dans le chloroforme est négatif.

**6.** Composé de la revendication 4 caractérisé en ce que Ar est du 4-trifluorométhylphényle.

**7.** Composé de la revendication 4 caractérisé en ce que Ar est du 4-trifluorométhoxyphényle.

**8.** Composé de la revendication 3 caractérisé en ce que Ar' est du 4-fluoro-3-phénoxyphényle ; Z est de l'oxygène ; et Ar est choisi parmi le 4-fluorophényle, le 3-chlorophényle, le 4-chlorophényle, le 4-bromophényle, le 4-méthylphényle, le 4-t-butylphényle, le 4-trifluorométhylphényle, le 4-méthoxyphényle, le 4-éthoxyphényle, le 4-(2-fluoroéthoxy)phényle, le 4-difluorométhoxyphényle, le 4-trifluorométhoxyphényle, et le 1,3-benzodioxol-5-yle.

**9.** Composé de la revendication 8 caractérisé en ce que Ar est du 4-chlorophényle.

**10.** Composé de la revendication 9 caractérisé en ce que $[\alpha]_D^{25}$ dans le chloroforme est négatif.

**11.** Composé de la revendication 8 caractérisé en ce que Ar est du 4-trifluorométhylphényle.

**12.** Composé de la revendication 8 caractérisé en ce que Ar est du 4-éthoxyphényle.

**13.** Composé de la revendication 8 caractérisé en ce que Ar est du 4-trifluorométhoxyphényle.

**14.** Composé de la revendication 3 caractérisé en ce que Ar' est du 2-méthyl[1,1'-biphényl]-3-yle ; Z est de l'oxygène ; et Ar est choisi parmi le phényle, le 4-fluorophényle, le 3-chlorophényle, le 4-chlorophényle, le 4-bromophényle, le 4-méthylphényle, le 4-t-butylphényle, le 4-trifluorométhylphényle, le 4-méthoxyphényle, le 4-éthoxyphényle, le 4-(2-fluoroéthoxy)phényle, le 4-difluorométhoxyphényle, le 4-trifluorométhoxyphényle, et le 1,3-benzodioxol-5-yle.

**15.** Composé de la revendication 14 caractérisé en ce que Ar est du 4-trifluorométhoxyphényle.

**16.** Composé de la revendication 3 caractérisé en ce que Ar est du 4-chlorophényle ; Z est du soufre ; et Ar' est choisi parmi le 3-phénoxyphényle, le 4-fluoro-3-phénoxyphényle, et le 2-méthyl[1,1'-biphényl]-3-yle.

**17.** Composé de la revendication 16 caractérisé en ce que Ar' est du 4-fluoro-3-phénoxyphényle.

**18.** Composé de la revendication 3 caractérisé en ce que Ar' est du 6-phénoxy-2-pyridyle, Z est de l'oxygène, et Ar est du 4-chlorophényle.

**19.** Composé de la revendication 3 caractérisé en ce que Ar' est choisi parmi le 3-phénoxyphényle, le 4-fluoro-3-phénoxyphényle, et le 2-méthyl[1,1'-biphényl]-3-yle ; Z est du méthyléne ; et Ar est choisi parmi le 3-chlorophényle, le 4-chlorophényle, le 4-méthylphényle, le 4-éthoxyphényle, et le 4-trifluorométhoxyphényle.

**20.** Composé de la revendication 19 caractérisé en ce que Ar' est du 3-phénoxyphényle et Ar est du 4-chlorophényle.

**21.** Composé de la revendication 19 caractérisé en ce que Ar' est du 3-phénoxyphényle et Ar est du 4-trifluorométhylphényle.

**22.** Composé de la revendication 19 caractérisé en ce que Ar' est du 4-fluoro-3-phénoxyphényle et Ar est du 4-chlorophényle.

**23.** Composé de la revendication 19 caractérisé en ce que Ar' est du 4-fluoro-3-phénoxyphényle et Ar est du 4-trifluorométhylphényle.

**24.** Composé de la revendication 19 caractérisé en ce que Ar' est du 4-fluoro-3-phénoxyphényle et Ar est

du 4-trifluorométhoxyphényle.

**25.** Composé de la revendication 19 caractérisé en ce que Ar' est du 2-méthyl[1,1'-biphényl]-3-yle et Ar est du 4-trifluorométhylphényle.

**26.** Composé caractérisé par la formule :

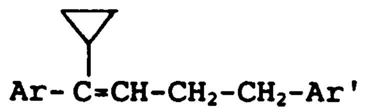

dans lequel Ar est un thiényle ou un phényle éventuellement substitué par un ou deux groupes choisis parmi un alkyle ($C_{1-6}$) à chaîne linéaire ou ramifiée, un halogène, un haloalkyle ($C_{1-4}$), un alkoxy ($C_{1-4}$), un haloalkoxy ($C_{1-4}$), ou un groupe de structure -A-$(CR^1R^2)_n$-A-, où $R^1$ et $R^2$ sont indépendamment, de l'hydrogène, un halogène, ou un alkyle ($C_{1-2}$), n est égal à 1 ou 2, et chaque A, qui peut être O, S, ou $CH_2$, est lié à un atome de carbone du cycle aromatique, les carbones auxquels sont fixés les groupes A étant adjacents les uns aux autres dans le cycle et Ar' est du 2-méthyl[1,1'-biphényl]-3-yle,du 3-phénoxyphényle, du 4-fluoro-3-phénoxyphényle, et du 6-phénoxy-2-pyridyle, dans lesquels le phényle, le pyridyle, ou le phénoxy peuvent éventuellement être substitués par un halogène ou un alkyle inférieur.

**27.** Composé de la revendication 26 caractérisé en ce que Ar' est choisi parmi le 3-phénoxyphényle et le 4-fluoro-3-phénoxyphényle et Ar est choisi parmi le 4-chlorophényle, le 4-bromophényle, et le 4-méthoxyphényle.

**28.** Composé de la revendication 27 caractérisé en ce que Ar' est du 4-fluoro-3-phénoxyphényle et Ar est du 4-chlorophényle.

**29.** Composé caractérisé par la formule :

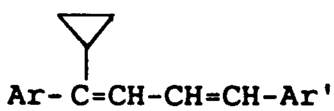

dans lequel Ar est un thiényle ou un phényle éventuellement substitué par un ou deux groupes choisis parmi un alkyle ($C_{1-6}$) à chaîne linéaire ou ramifiée, un halogène, un haloalkyle ($C_{1-4}$), un alkoxy ($C_{1-4}$), un haloalkoxy ($C_{1-4}$), ou un groupe de structure -A-$(CR^1R^2)_n$-A-, où $R^1$ et $R^2$ sont indépendamment, de l'hydrogène, un halogène, ou un alkyle ($C_{1-2}$), n est égal à 1 ou 2, et chaque A, qui peut être O, S, ou $CH_2$, est lié à un atome de carbone du cycle aromatique, les carbones auxquels sont fixés les groupes A étant adjacents les uns aux autres dans le cycle et Ar' est du 2-méthyl[1,1'-biphényl]-3-yle,du 3-phénoxyphényle, du 4-fluoro-3-phénoxyphényle, et du 6-phénoxy-2-pyridyle, dans lesquels le phényle, le pyridyle, ou le phénoxy peuvent éventuellement être substitués par un halogène ou un alkyle inférieur.

**30.** Composé de la revendication 29 caractérisé en ce que Ar' est choisi parmi le 3-phénoxyphényle, le 4-fluoro-3-phénoxyphényle, et le 2-méthyl[1,1'-biphényl]-3-yle et Ar est choisi parmi le 4-chlorophényle, le 4-méthylphényle, le 4-trifluorométhylphényle, et le 4-éthoxyphényle.

**31.** Composition insecticide ou acaricide caractérisée par une quantité efficace contre les insectes et les acariens d'un composé de la revendication 1 en mélange avec un ou plusieurs supports agricoles compatibles, diluants, adjuvants ou pesticides complémentaires.

**32.** Composition insecticide caractérisée par une quantité efficace contre les insectes d'un composé de la revendication 26 en mélange avec un ou plusieurs supports agricoles compatibles, diluants, adjuvants ou pesticides complémentaires.

**33.** Composition insecticide caractérisée par une quantité efficace contre les insectes d'un composé de la revendication 29 en mélange avec un ou plusieurs supports agricoles compatibles, diluants, adjuvants ou pesticides complémentaires.

**34.** Méthode de contrôle des insectes et des acariens caractérisée par l'application à l'endroit où le contrôle est désiré d'une quantité efficace contre les insectes ou les acariens d'un composé de la revendication 1.

**35.** Méthode de contrôle des insectes caractérisée par l'application à l'endroit où le contrôle est désiré d'une quantité efficace contre les insectes d'un composé de la revendication 26.

**36.** Méthode de contrôle des insectes caractérisée par l'application à l'endroit où le contrôle est désiré d'une quantité efficace contre les insectes d'un composé de la revendication 29.

**37.** Procédé de préparation d'un composé de formule :

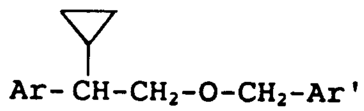

dans lequel Ar est un thiényle ou un phényle éventuellement substitué par un ou deux groupes choisis parmi un alkyle ($C_{1-6}$) à chaîne linéaire ou ramifiée, un halogène, un haloalkyle ($C_{1-4}$), un alkoxy ($C_{1-4}$), un haloalkoxy ($C_{1-4}$), ou un groupe de structure $-A-(CR^1R^2)_n-A-$, où $R^1$ et $R^2$ sont indépendamment, de l'hydrogène, un halogène, ou un alkyle ($C_{1-2}$), n est égal à 1 ou 2, et chaque A, qui peut être O, S, ou $CH_2$, est lié à un atome de carbone du cycle aromatique, les carbones auxquels sont fixés les groupes A étant adjacents les uns aux autres dans le cycle et Ar' est du 2-méthyl[1,1'-biphényl]-3-yle,du 3-phénoxyphényle, du 4-fluoro-3-phénoxyphényle, et du 6-phénoxy-2-pyridyle, dans lesquels le phényle, le pyridyle, ou le phénoxy peuvent éventuellement être substitués par un halogène ou un alkyle inférieur, caractérisé en ce qu'un composé de formule

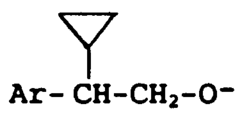

entre en réaction avec un composé de formule,

$Ar'-CH_2-X$

où X est un groupe qui part capable d'être déplacé par des ions éthoxyde.

**38.** Procédé de préparation d'un composé de formule

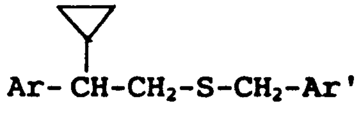

dans lequel Ar est un phényle éventuellement substitué par un ou deux groupes choisis parmi un alkyle ($C_{1-6}$) à chaîne linéaire ou ramifiée, un halogène, un haloalkyle ($C_{1-4}$), un alkoxy ($C_{1-4}$), un haloalkoxy ($C_{1-4}$), ou un groupe de structure $-A-(CR^1R^2)_n-A-$, où $R^1$ et $R^2$ sont indépendamment, de l'hydrogène, un halogène, ou un alkyle ($C_{1-2}$), n est égal à 1 ou 2, et chaque A, qui peut être O, S, ou $CH_2$, est lié à un atome de carbone du cycle aromatique, les carbones auxquels sont fixés les groupes A étant adjacents les uns aux autres dans le cycle et Ar' est du 2-méthyl[1,1'-biphényl]-3-yle, du 3-phénoxyphényle, du 4-fluoro-3-phénoxyphényle, et du 6-phénoxy-2-pyridyle, dans lesquels le phényle, le pyridyle, ou le phénoxy peuvent éventuellement être substitués par un halogène ou un alkyle inférieur, caractérisé en ce qu'un composé de formule

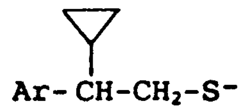

entre en réaction avec un composé de formule,

Ar'-$CH_2$-X

où X est un groupe qui part capable d'être déplacé par des ions thioéthoxyde.

**39.** Procédé de préparation d'un composé de formule

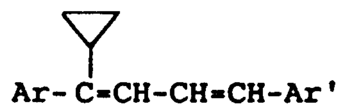

dans lequel Ar est un phényle éventuellement substitué par un ou deux groupes choisis parmi un alkyle ($C_{1-6}$) à chaîne linéaire ou ramifiée, un halogène, un haloalkyle ($C_{1-4}$), un alkoxy ($C_{1-4}$), un haloalkoxy ($C_{1-4}$), ou un groupe de structure -A-$(CR^1R^2)_n$-A-, où $R^1$ et $R^2$ sont indépendamment, de l'hydrogène, un halogène, ou un alkyle ($C_{1-2}$), n est égal à 1 ou 2, et chaque A, qui peut être O, S, ou $CH_2$, est lié à un atome de carbone du cycle aromatique, les carbones auxquels sont fixés les groupes A étant adjacents les uns aux autres dans le cycle et Ar' est du 2-méthyl[1,1'-biphényl]-3-yle, du 3-phénoxyphényle, du 4-fluoro-3-phénoxyphényle, et du 6-phénoxy-2-pyridyle, dans lesquels le phényle, le pyridyle, ou le phénoxy peuvent éventuellement être substitués par un halogène ou un alkyle inférieur, caractérisé en ce qu'un composé de formule

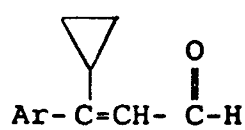

entre en réaction avec un composé de formule,

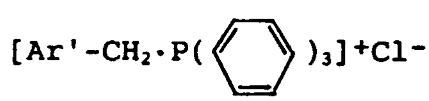

**40.** Procédé de préparation d'un composé de formule

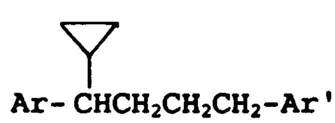

dans lequel Ar est un thiényle ou un phényle éventuellement substitué par un ou deux groupes choisis parmi un alkyle ($C_{1-6}$) à chaîne linéaire ou ramifiée, un halogène, un haloalkyle ($C_{1-4}$), un alkoxy ($C_{1-4}$), un haloalkoxy ($C_{1-4}$), ou un groupe de structure -A-$(CR^1R^2)_n$-A-, où $R^1$ et $R^2$ sont indépendamment, de l'hydrogène, un halogène, ou un alkyle ($C_{1-2}$), n est égal à 1 ou 2, et chaque A, qui peut être

O, S, ou $CH_2$, est lié à un atome de carbone du cycle aromatique, les carbones auxquels sont fixés les groupes A étant adjacents les uns aux autres dans le cycle et Ar' est du 2-méthyl[1,1'-biphényl]-3-yle,du 3-phénoxyphényle, du 4-fluoro-3-phénoxyphényle, et du 6-phénoxy-2-pyridyle, dans lesquels le phényle, le pyridyle, ou le phénoxy peuvent éventuellement être substitués par un halogène ou un alkyle inférieur, caractérisé en ce qu'un composé de formule

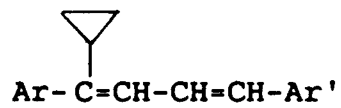

est hydrogéné.

**41.** Procédé de préparation d'un composé de formule

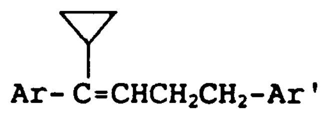

dans lequel Ar est un phényle éventuellement substitué par un ou deux groupes choisis parmi un alkyle $(C_{1-6})$ à chaîne linéaire ou ramifiée, un halogène, un haloalkyle $(C_{1-4})$, un alkoxy $(C_{1-4})$, un haloalkoxy $(C_{1-4})$, ou un groupe de structure $-A-(CR^1R^2)_n-A-$, où $R^1$ et $R^2$ sont indépendamment, de l'hydrogène, un halogène, ou un alkyle $(C_{1-2})$, n est égal à 1 ou 2, et chaque A, qui peut être O, S, ou $CH_2$, est lié à un atome de carbone du cycle aromatique, les carbones auxquels sont fixés les groupes A étant adjacents les uns aux autres dans le cycle et Ar' est du 2-méthyl[1,1'-biphényl]-3-yle, du 3-phénoxyphényle, du 4-fluoro-3-phénoxyphényle, et du 6-phénoxy-2-pyridyle, dans lesquels le phényle, le pyridyle, ou le phénoxy peuvent éventuellement être substitués par un halogène ou un alkyle inférieur, caractérisé en ce qu'un composé de formule

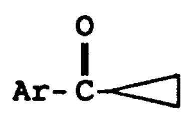

entre en réaction avec un composé de formule

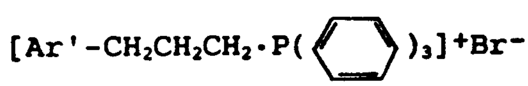

**42.** Procédé de préparation d'un composé de formule

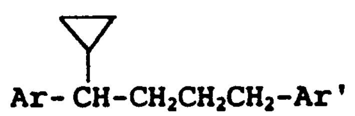

dans lequel Ar est un phényle éventuellement substitué par un ou deux groupes choisis parmi un alkyle $(C_{1-6})$ à chaîne linéaire ou ramifiée, un halogène, un haloalkyle $(C_{1-4})$, un alkoxy $(C_{1-4})$, un haloalkoxy $(C_{1-4})$, ou un groupe de structure $-A-(CR^1R^2)_n-A-$, où $R^1$ et $R^2$ sont indépendamment, de l'hydrogène, un halogène, ou un alkyle $(C_{1-2})$, n est égal à 1 ou 2, et chaque A, qui peut être O, S, ou $CH_2$, est lié à un atome de carbone du cycle aromatique, les carbones auxquels sont fixés les groupes A étant adjacents les uns aux autres dans le cycle et Ar' est du 2-méthyl[1,1'-biphényl]-3-yle, du 3-phénoxyphényle, du 4-fluoro-3-phénoxyphényle, et du 6-phénoxy-2-pyridyle, dans lesquels le phényle, le

pyridyle, ou le phénoxy peuvent éventuellement être substitués par un halogène ou un alkyle inférieur, caractérisé en ce qu'un composé de formule

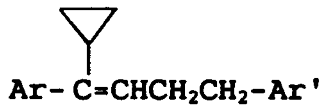

est hydrogéné.